# EUROPEAN PATENT SPECIFICATION

(11) **EP 1 541 575 B1**
(45) Date of publication and mention of the grant of the patent: **03.03.2010**
(21) Application number: 03771268.4
(22) Date of filing: 16.07.2003
(51) Int. Cl.: C07D 487/04, A01N 47/36

(54) **NOVEL PROCESS FOR PRODUCING IMIDAZO(1,2-B)PYRIDAZINE DERIVATIVE**
NEUES VERFAHREN ZUR HERSTELLUNG EINES IMIDAZO(1,2-B)PYRIDAZINDERIVATS
NOUVEAU PROCEDE POUR PRODUIRE UN DERIVE D'IMIDAZO(1,2-B)PYRIDAZINE

(30) Priority: 29.07.2002 JP 2002219786; 15.01.2003 WO PCT/JP03/00244; 26.03.2003 JP 2003085617
(43) Date of publication of application: 15.06.2005
(73) Proprietor: Sumitomo Chemical Company, Limited, Chuo-ku Tokyo (JP)
(72) Inventor: TABUCHI, Takanori, Osaka-shi, Osaka (JP); YAMAMOTO, Tetsuhiro, Nishinomiya-shi, Hyogo (JP); KAJIWARA, Takeshi, Hyogo 665-0061 (JP)
(74) Representative: von Kreisler, Alek
(86) International application number: PCT/JP2003/009003
(87) International publication number: WO 2004/011466

(56) References cited:
- WO-A1-00/23450
- WO-A1-03/061388
- JP-A- 1 316 379
- TAMAO K ET AL: "NICKEL-PHOSPHINE COMPLEX-CATALYZED GRIGNARD COUPLING. I. CROSS-COUPLING OF ALKYL, ARYL, AND ALKENYL GRIGNARD REAGENTS WITH ARYL AND ALKENYL HALIDES: GENERAL SCOPE AND LIMITATIONS" BULLETIN OF THE CHEMICAL SOCIETY OF JAPAN, CHEMICAL SOCIETY OF JAPAN, TOKYO, JP, vol. 49, no. 7, July 1976 (1976-07), pages 1958-1969, XP000996380 ISSN: 0009-2673
- MOURAD A.E. ET AL.: 'Methyl Imidazo (1,2-b) pyridazine-2-carbamates and related compounds as potential antifilarial agents' J. HETEROCYCLIC CHEM. vol. 29, 1992, pages 1583 - 1592, XP002973586
- POLLAK A. ET AL.: 'Synthesis of pyridazine derivatives' TETRAHEDRON vol. 24, no. 6, 1968, pages 2623 - 2629, XP002973587
- ISHIKAWA T. ET AL.: 'Studies on anti-MRSA parenteral cephalosporins' THE JOURNAL OF ANTIBIOTICS vol. 54, no. 3, 2001, pages 257 - 277, XP001056921
- SATOH K. ET AL.: 'Reactivity of imidazo (1,2-b), pyridazine 1-oxide' HETEROCYCLES vol. 10, 1978, pages 269 - 276, XP002973588

## Description

### Technical Field

This invention relates to a process for producing an imidazo[1,2-b]pyridazine derivative having a substituent bonded to the 6-position carbon atom which is used for producing a sulfonylurea compound having a fused heterocyclic ring useful as a herbicide, and an intermediate thereof.

### Background Art

Sulfonylurea compounds having a fused heterocyclic ring are known as a herbicide having a high herbicidal activity and high safety for crops (e.g., see JP-B H05-36439 and JP-A H01-139582). Among them, a sulfonylurea compound having imidazo[1,2-b]pyridazine ring as a fused heterocyclic ring is one of compound groups having a high activity, especially the present inventors found out that a compound having a substituent bonded to the 6-position carbon atom of imidazo[1,2-b]pyridazine ring has a high herbicidal activity against weeds resistant to conventional sulfonylurea herbicides, and filed a patent application (JP Application No. 2003-6756). As a reaction for introducing a substituent at the 6-position carbon atom of imidazo[1,2-b]pyridazine ring, Journal of Antibiotics, 54(3), 257-277, 2001; Synthesis, (4), 595-600, 2001; JP-A H05-271233; JP-A H06-116272; JP-A H11-310581; JP-A H11-310582; JP-A 2000-198735; JP-A 2001-199889 and the like are known. MOURAD A.E. ET AL.: 'Methyl Imidazo (1,2-b) pyridazine-2-carbamates and related compounds as potential antifilarial agents' J. HETEROCYCLIC CHEM. vol. 29, 1992, pages 1583 - 1592, XP002973586 relates to compounds of formula II of the present invention wherein R is CH₃, Y is H and X is t-Butyl. It also discloses a method of synthesis of the said compounds from aminopyridazines.

POLLAK A. ET AL.: 'Synthesis of pyridazine derivatives' TETRAHEDRON vol. 24, no. 6, 1968, pages 2623 - 2629, XP002973587 relates to compounds of formula II of the present application wherein R₁ = Me and R₂ = H. It also discloses a method of synthesis of the said compounds from aminopyridazines.

The object of this invention is to provide a process for easily and inexpensively producing an imidazo[1,2-b]pyridazin-3-ylsulfonamide derivative having a substituent bonded to the 6-position carbon atom which is useful as a synthetic intermediate for herbicides.

### Disclosure of Invention

To solve the above-mentioned problem, the present inventors made extensive study to find out an easy and inexpensive production process of imidazo[1,2-b]pyridazin-3-ylsulfonamide compound having a substituent bonded to the 6-position carbon atom over the years, and as a result, found out that an imidazo[1,2-b]pyridazine derivative having a substituent bonded to the 6-position carbon atom can be obtained unexpectedly with a convenient operation and in good yield by reacting an imidazo[1,2-b]pyridazine derivative having a leaving group at the 6-position with an organometallic compound under the presence of transition metal catalyst. After further studying extensively based on these knoweldge, this invention was thereby completed. That is, this invention relates to:
(1) A process for producing a compound represented by the formula (II): 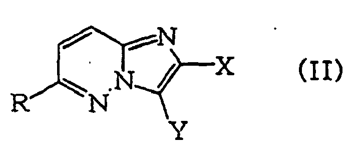
   wherein X represents a chlorine atom,
   Y represents a hydrogen atom or SO₂N=CH-NR¹R² (wherein R¹ and R² represent each lower alkyl group, or R¹ and R² may be combined together with the adjacent nitrogen atom to form a heterocyclic ring), and R represents a C₁₋₆ alkyl group, C₃₋₇ cycloalkyl group which may be substituted with C₁₋₆ alkyl, C₂₋₆ alkenyl group or C₂₋₆ alkynyl group, which comprises reacting an imidazo[1,2-b]pyridazine compound represented by the formula (I): 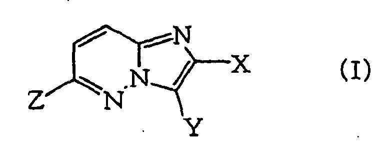
   wherein X and Y are as defined above, and Z represents a chlorine atom , with one or more compounds selected from the organometallic compounds represented by the formula: 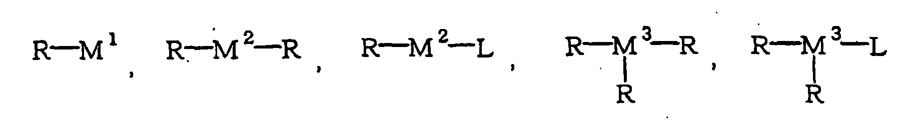 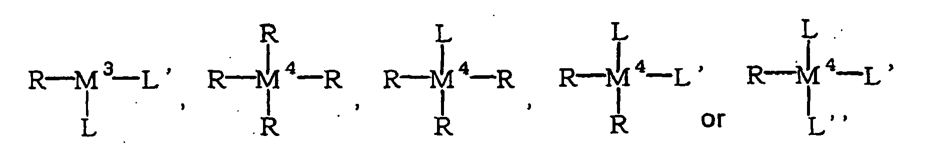
   wherein R is as defined above, and M¹ represents an univalent metal, M² represents a divalent metal, M³ represents a trivalent metal and M⁴ represents a tetravalent metal, and L, L' and L" are the same or different and represent an anion, under the presence of transition metal catalyst.
(2) The process according to the abovementioned (1), wherein the metal of the transition metal catalyst is palladium, nickel or iron.
(3) The process according to the abovementioned (1), wherein the metal of the transition metal catalyst is nickel.
(4) The process according to the abovementioned (1) wherein the metal of the organometallic compound is magnesium or zinc.
(5) The process according to the abovementioned (1), wherein R is a C₁₋₆ alkyl group or C₃₋₇ cycloalkyl group which may be substituted with C₁₋₆ alkyl.
(6) The process according to the abovementioned (1), wherein Y is a hydrogen atom and R is a C₁₋₆ alkyl group.
(7) The process according to the abovementioned (3), wherein the metal of the organometallic compound is magnesium or zinc.
(8) The process according to the abovementioned (7), wherein the organometallic compound is a C₁₋₆ alkylmagnesium halide or a C₁₋₆, alkylzinc halide.
(9) The process according to the abovementioned (8), wherein the organometallic compound is a propylmagnesium halide or propylzinc halide and the nickel catalyst is [1,3-bis(diphenylphosphino)propane]nickel(II) dichloride or bis(triphenylphosphine)nickel(II) dichloride.
(10) A process for producing a sulfonamide compound represented by the formula (III): 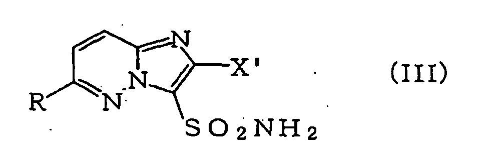 wherein X represents a halogen atom or an optionally halogenated C₁₋₆ alkyl group and R represents a C₁₋₆ alkyl group, C₁₋₆ cycloalkyl group which may be substituted with C₁₋₆ alkyl, C₂₋₆ alkenyl group or C₂₋₆ alkynyl group, which comprises sulfonating with chlorosulfonic acid a compound represented by the formula (IIa): 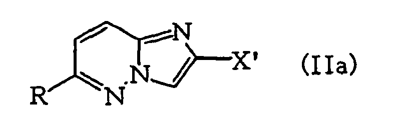 wherein X' and R are as defined above, which is obtained by reacting an imidazo[1,2-b]pyridazine compound represented by the formula (Ia): 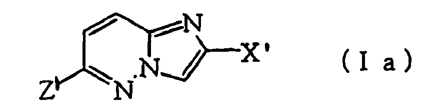 wherein X' is as defined above, and Z' represents a halogen atom or OSO₂R³ (wherein R³ represents an optionally fluorinated C₁₋₆ alkyl group or phenyl group which may be substituted with C₁₋₆ alkyl), with one or more compounds selected from the organometallic compounds represented by the formula: 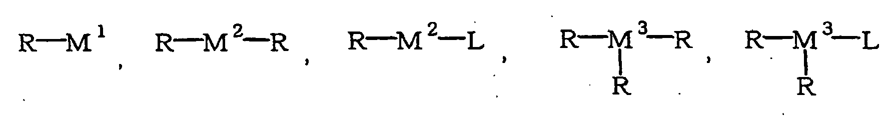 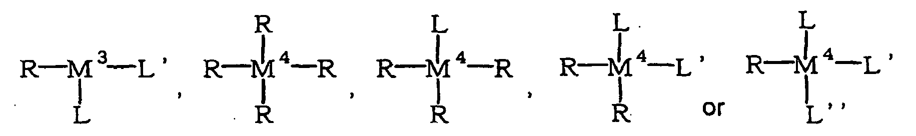 wherein R is as defined above, and M¹ represents an univalent metal, M² represents a divalent metal, M³ represents a trivalent metal and M⁴ represents a tetravalent metal, and L, L' and L" are the same or different and represent an anion, under the presence of transition metal catalyst, followed by converting to a sulfonyl chloride with phosphorus oxychloride, then reacting with ammonia.

### Mode for Carrying Out the Invention

The present invention will be described in detail below.

Given the term alkyl group, alkenyl group, alkynyl group etc. in this specification, these alkyl group and the like are meant to be composed of 1 or 2 to 6 carbon atoms, preferably 1 or 2 to 4 carbon atoms. For example, a linear or branched C₁₋₆ alkyl group, C₂₋₆ alkenyl group and C₂₋₆ alkynyl group are exemplified. In addition, the "cycloalkyl group" means C₃₋₇ cycloalkyl group having 3 to 7 carbon atoms.

In the above-mentioned formulas I and II X represents a chlorine atom, X' in formulas Ia, Ib, IIa, IIb and III represents a halogen atom or an optionally halogenated alkyl group, and the "halogen atom" in "halogen atom" and "optionally halogenated alkyl group" includes, for example, fluorine, chlorine, bromine, iodine Examples of the "alkyl group" in "optionally halogenated alkyl group" include C₁₋₆ alkyl group such as methyl, ethyl, propyl, isopropyl, butyl, isobutyl, s-butyl, t-butyl. Examples of preferable substituent as X' include fluorine, chlorine, methyl, ethyl, trifluoromethyl.

When R¹ and R² of SO₂N=CH-NR¹R² for Y and Y' in the above formulas (I) and (Ib) represent independently a alkyl group, the "alkyl group" includes, for example, C₁₋₆ alkyl group such as methyl, ethyl, propyl, isopropyl, butyl, isobutyl, s-butyl, t -butyl, and the "heterocyclic ring" in case that "R¹ and R² are combined together with the adjacent nitrogen atom to form a heterocyclic ring" includes, for example, a 3- to 10-membered (preferably, 3- to 6-membered) nitrogen-containing heterocyclic ring such as azetidine ring, pyrrolidine ring, piperidine ring.

In the above-mentioned formulas I and II Z represents a chlorine atom in formulas Ia and Ib Z' represents a "halogen atom" for example, fluorine, chlorine, bromine, iodine etc. The "alkyl group" in the "optionally fluorinated alkyl group" for R³ of "OSO₂R³" includes, for example, C₁₋₆ alkyl group such as methyl, ethyl, propyl, isopropyl, butyl, isobutyl, s-butyl, t-butyl. The "alkyl group" of the "phenyl group which may be substituted with alkyl" includes, for example, C₁₋₆ alkyl group such as methyl, ethyl, propyl, isopropyl, butyl, isobutyl, s-butyl, t-butyl. Examples of preferable substituent as Z include a halogen atom, in particular, chlorine and bromine are preferred.

In the organometallic compounds represented by the formula: 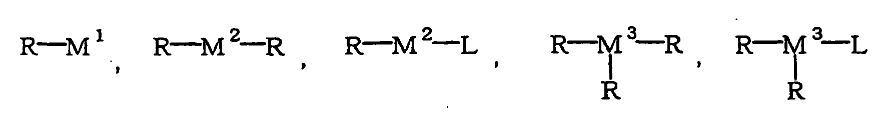 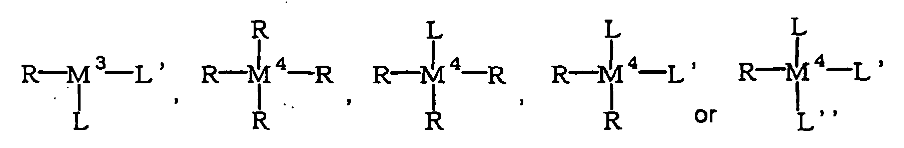 and formulas (II), (IIa) and (IIb), examples of the "alkyl group" in the "alkyl group" and "cycloalkyl group which may be substituted with alkyl" for R include C₁₋₆ alkyl group such as methyl, ethyl, propyl, isopropyl, butyl, isobutyl, s-butyl, t-butyl. Examples of the "cycloalkyl group" in the "cycloalkyl group which may be substituted with alkyl" include C₃₋₇ cycloalkyl such as cyclopropyl, cyclobutyl, cyclopentyl, etc. Examples of the "alkenyl group" include C₂₋₆ alkenyl such as ethenyl, 1-propenyl, 2-propenyl, 1-butenyl, 2-butenyl, 3-butenyl, and examples of the "alkynyl group" include C₂₋₆ alkynyl such as ethynyl, 1-propynyl, 2-propynyl, 2-butynyl. The preferred substituent as R includes ethyl, propyl, isopropyl and cyclopropyl.

As M¹ which represents an univalent metal in the above-mentioned organometallic compounds, lithium, sodium, potassium, rubidium, univalent copper are exemplified. M² which represents a divalent metal include, for example, magnesium, calcium, strontium, barium, zinc, cadmium, mercury, divalent copper, divalent lanthanoid metal and the like. M³ which represents a trivalent metal include, for example, boron, aluminum, trivalent lanthanoid metal. M⁴ which represents a tetravalent metal include, for example, silicon, germanium, tin, lead, titanium, zirconium, cerium. Preferred metal is an univalent or divalent metal, and in particular, magnesium or zinc is preferred.

The anions represented by L, L' or L" in the above-mentioned organometallic compounds are the same or different and include, for example, a halogen such as fluorine, chlorine, bromine, iodine, C₁₋₆ alkoxy group such as methoxy, ethoxy, propoxy, phenoxy group, C₁₋₆ alkyl group such as methyl, ethyl, propyl, isopropyl, butyl etc., carboxylic acid anion such as acetate, trifluoroacetate, benzoate, phenyl group, cyano group, hydroxy group, or two of L, L' or L" may be combined together to form a dialkoxide of diol such as ethylene glycol and catechol. Preferred is a halogen.

Preferable examples of the organometallic compound include organic alkalimetallic compound, organic alkaline earth metallic compound, organozinc compound, organocupric compound, organosilicon compound and organolead compound, and in particular, preferred are organomagnesium halide and organozinc halide.

The organometallic compound is usually commercially available or prepared from a halide such as alkyl halide, cycloalkyl halide, alkenyl halide, alkynyl halide, and a metal simple substance, or it can be obtained by a metal exchange reaction of easily available organometallic compound and other metal salt. Examples thereof include a preparation of organozinc compound by a reaction of organolithium compound or organomagnesium compound with zinc chloride, a preparation of organotitanium compound by a reaction of organolithium compound or organomagnesium compound with titanium chloride, a preparation of organocerium compound by a reaction of organolithium compound or organomagnesium compound with cerium chloride, a preparation of organocopper compound by a reaction of organolithium compound or organomagnesium compound with copper chloride.

Furthermore, the organometallic compound can be used with generating it from a halide such as alkyl halide, cycloalkyl halide, alkenyl halide, alkynyl halide, and a metal simple substance in the system of coupling reaction with imidazo[1,2-b]pyridazines.

Examples of the transition metal catalyst include a transition metal simple substance, a catalyst wherein transition metal is fixed on a carrier, a transition metal complex, a polymerized transition metal, a transition metal complex which is fixed in a microcapsule. Examples of the transition metal include titanium, vanadium, chromium, manganese, iron, cobalt, nickel, cupper, zirconium, niobium, molybdenum, ruthenium, rhodium, palladium, silver, hafnium, tantalum, tungsten, rhenium, osmium, iridium, platinum, gold, and preferred is palladium, nickel or iron, and in particular, nickel is preferred.

In the case of complex, examples of ligand include a halogen anion such as fluorine anion, chlorine anion, bromine anion, iodine anion, cyano anion, alkoxy anion such as methoxy, ethoxy, isopropoxy, carboxylic acid anion such as acetate anion, trifluoroacetate anion, sulfonic acid anion such as methanesulfonate, trifluoromethanesulfonate, p-toluenesulfonate, amines such as ammonia, methylamine, ethylamine, dimethylamine, ethylenediamine, triethylamine, aniline, N,N-dimethylaniline, pyridine, 2,2'-bipyridyl, imidazole, alkoxides of aminoalcohol such as ethanolamine, propanolamine, phosphines such as tributylphosphine, tricyclohexylphosphine, triphenylphosphine, 1,2-bis(diphenylphosphino)ethane, 1,3-bis(diphenylphosphino)propane, 1,4-bis(diphenylphosphino)butane, aminoalkylphosphines such as 2-(dimethylamino)ethyldiphenylphosphine, alkoxides of hydroxyalkylphosphine such as diphenyl(2-hydroxyethyl)phosphine, carbon monoxide, ethylene, butadiene, cyclopentadienyl anion, 1,5-cyclooctadiene, acetonitrile, benzonitrile, acetylacetonate anion, dibenzalacetone. The transition metal complex is composed of same or different 1 to 6 ligands selected from the above-mentioned ligands.

The transition metal complex is preferably a palladium or nickel complex containing phosphines as a ligand such as bis(triphenylphosphine)nickel(II) dichloride, bis(triphenylphosphine)nickel(II) dibromide, [1,3-bis(diphenylphosphino)propane]nickel(II) dichloride, [1,3-bis(diphenylphosphino)propane]nickel(II) dibromide, bis(triphenylphosphine)palladium(II) dichloride, bis(triphenylphosphine)palladium(II) dibromide, [1,3-bis(diphenylphosphino)propane]palladium(II) dichloride, [1,3-bis(diphenylphosphino)propane]palladium(II) dibromide and tetrakis(triphenylphosphine)palladium, or an iron compound such as iron(II) chloride, iron(III) chloride or iron(III) acetylacetonate, and particularly preferred are bis(triphenylphosphine)nickel(II) dichloride, [1,2-bis(diphenylphosphino)ethane]nickel(II) dichloride and [1,3-bis(diphenylphosphino)propane]nickel(II) dichloride.

The transition metal complex is usually commercially available or prepared by a known method, or the transition metal complex can be used with generating it in the coupling reaction system by adding a transition metal salt and a ligand separately. Examples thereof include a combination of nickel chloride and triphenylphosphine, a combination of nickel bromide and triphenylphosphine, a combination of nickel acetate and triphenylphosphine, a combination of palladium chloride and triphenylphosphine, a combination of palladium bromide and triphenylphosphine, a combination of palladium acetate and triphenylphosphine, a combination of nickel chloride and 1,2-bis(diphenylphosphino)ethane, a combination of nickel bromide and 1,2-bis(diphenylphosphino)ethane, a combination of nickel acetate and 1,2-bis(diphenylphosphino)ethane, a combination of palladium chloride and 1,2-bis(diphenylphosphino)ethane, a combination of palladium bromide and 1,2-bis(diphenylphosphino)ethane, a combination of palladium acetate and 1,2-bis(diphenylphosphino)ethane, a combination of nickel chloride and 1,3-bis(diphenylphosphino)propane, a combination of nickel bromide and 1,3-bis(diphenylphosphino)propane, a combination of nickel acetate and 1,3-bis(diphenylphosphino)propane, a combination of palladium chloride and 1,3-bis(diphenylphosphino)propane, a combination of palladium bromide and 1,3-bis(diphenylphosphino)propane, a combination of palladium acetate and 1,3-bis(diphenylphosphino)propane.

The reaction in the production of compound (II) from compound (I) is carried out without solvent or with making a dilution in solvent. Examples of the reaction solvent include hydrocarbon solvents such as petroleum ether, pentane, hexane, cyclohexane, benzene, toluene, xylene, halogenated hydrocarbon solvents such as dichloromethane, 1,2-dichloroethane, chloroform, carbon tetrachloride, trichloroethane, trichloroethylene, tetrachloroethane, chlorobenzene, ether solvents such as diethyl ether, methyl t-butyl ether, tetrahydrofuran (abbreviation: THF), 1,4-dioxane, dimethoxyethane (abbreviation: DME), diethylene glycol dimethyl ether, ketone solvents such as acetone, methyl ethyl ketone, 2-pentanone, 3-pentanone, cyclohexanone, ester solvents such as ethyl acetate, butyl acetate, amide solvents such as N,N-dimethylformamide (abbreviation: DMF), N,N-dimethylacetamide, N-methylpyrrolidone, nitrile solvents such as acetonitrile, propionitrile, solvents containing sulfur such as dimethyl sulfoxide, dimethyl sulfone, solfolane, carbon disulfide, nitro compound solvents such as nitromethane, nitrobenzene, protic solvents such as water, methanol, ethanol, propanol, isopropanol, t-butanol, ethylene glycol, phenol, acetic acid. As a preferable solvent, hydrocarbon solvents or ether solvents are exemplified. These solvents are usually used alone, or may be used with mixing in an appropriate ratio.

The reaction temperature is -100°C to 300°C, preferably -50°C to 100°C, and more preferably -20°C to 50°C.

The reaction time is 10 seconds to 500 hours, preferably 1 minute to 48 hours, and more preferably 10 minutes to 24 hours.

The ratio of the organometallic compound used to compound (I) is 0.5 to 10 equivalents, preferably 0.8 to 3.0 equivalents, and particularly preferably 1.0 to 1.5 equivalents.

The ratio of the transition metal catalyst used to compound (I) is 0.000001 to 10 equivalents, preferably 0.00001 to 1 equivalent, and particularly preferably 0.0001 to 0.1 equivalent.

The method which leads compound (IIa) to a sulfonamide represented by the formula (III) by sulfonating compound (IIa) with chlorosulfonic acid, followed by converting to sulfonyl chloride with phosphoryl chloride, and then reacting with ammonia, can be carried out according to a similar method to the known art (JP-B H05-36439).

The method which leads compound (IIb) to a sulfonamide represented by the formula (III) by hydrolyzing compound (IIb) can be carried out according to a similar method to the known art (Protective Groups in Organic Synthesis, page 275) .

The reaction of the present invention has a feature that the substituent Z of 6-position is selectively substituted even when the substituent X of 2-position of imidazo[1,2-b]pyridazine ring is a halogen atom such as chlorine atom etc. Furthermore, the reaction of the present invention has a feature that the reaction proceeds without amide polar solvents such as HMPT (hexamethylphosphoric triamide) and DMA (dimethylacetamide) used in the prior art, which is suspected of toxicity. In addition, the reaction of the present invention proceeds at ice-cooling to room temperature in most cases with the exception of using palladium catalyst in toluene solvent, and heating such as disclosed in prior arts is not required.

### Examples

Hereinafter, this invention will be further illustrated by Examples and Reference Examples, but this invention is not limited thereto. The elution in silica gel column chromatography was carried out under the observation by TLC (Thin Layer Chromatograph). In the observation by TLC, kieselgel 60F254 (70 to 230 mesh) manufactured by Merck was used as TLC plate, the solvent used as an eluting solvent in column chromatography was used as developing solvent, and a UV detector or iodine color-developing method was used for detection. As silica gel for column, kieselgel 60 (70 to 230 mesh) manufactured by Merck was used. In case that a mixed solvent was used as developing solvent, the numeric value shown in parentheses indicates volumetric mixing ratio of each solvents. NMR (nuclear magnetic resonance) spectra show proton NMR, and were determined with Bruker AV-400 (400 MHz) spectrometer with tetramethylsilane as internal standard, and all delta values are shown in ppm. The abbreviations used in the following Reference Examples and Examples have the following meanings. s: singlet, d: doublet, t: triplet, q: quartet, m: multiplet, dd: double doublet, dt: double triplet, dq: double quartet, sept: septet, br: broad, brs: broad singlet, ddd: double double doublet, ddt: double double triplet, brd: broad doublet, brq: broad quartet, J: coupling constant, Hz: Hertz, Me: methyl group, Et: ethyl group, Pr: propyl group, i-Pr: isopropyl group, c-Pr: cyclopropyl group, Bu: butyl group, i-Bu: isobutyl group, dppp: 1,3-bis(diphenylphosphino)propane, PPh₃: triphenylphosphine, CDCl₃: heavy chloroform, DMSO-d₆: heavy dimethyl sulfoxide, DMF: N,N-dimethylformamide, HPLC: high performance liquid chromatography, %: weight %, mp: melting point, and room temperature means a temperature of 15 to 25°C.

### Example 1

### Synthesis of 6-ethyl-2-methylimidazo[1,2-b]pyridazine

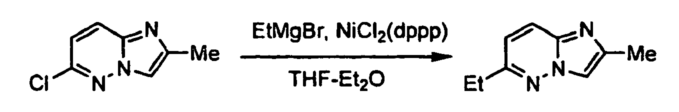

6-Chloro-2-methylimidazo[1,2-b]pyridazine (5.00 g, 29.8 mmol) and [1,3-bis(diphenylphosphino)propane]nickel(II) dichloride (0.08 g, 0.15 mmol) were suspended in dry ether (40 ml)-dry THF (20 ml), and a solution of ethylmagnesium bromide in ether (3 M, 15 ml, 45 mmol) was added dropwise thereto with stirring under ice-cooling over 5 minutes (internal temperature 10°C or less). The temperature of the reaction solution was increased to room temperature, and the mixture was stirred at the same temperature for 2 hours and under reflux with heating for 3 hours. The reaction solution was left to cool to room temperature under stirring, and water (30 ml) was added little by little. Further, the pH of reaction mixture was adjusted to about 5 to 6 with concentrated hydrochloric acid under stirring at room temperature. The organic layer and the aqueous layer were separated from each other, and the aqueous layer was extracted with ethyl acetate (70 mlx2). The organic layers were combined and washed with water (250 mlx3). The organic layer was dried over magnesium sulfate and concentrated, and the residues were purified by silica gel column chromatography (chloroform : ethyl acetate = 2 : 1 → 1 : 1), and the resulting crude oil was further purified by silica gel column chromatography (ethyl acetate), and the title compound was obtained as pale red oil. The yield was 1.32 g (27.4%).
¹H NMR (CDCl₃, δ): 1.33 (3H,t,J=7.5 Hz), 2.48 (3H,s), 2.82 (2H,q,J=7.5 Hz), 6.87 (1H,d,J=9.2 Hz), 7.65 (1H,s), 7.72 (1H,d,J=9.2 Hz)
IR (Neat, cm⁻¹): 2973, 2934, 2876, 1543, 1460, 1382, 1333, 1300, 1263, 1155, 1125, 1057, 1000, 820, 726, 699

### Example 2

### Synthesis of 6-ethyl-2-methylimidazo[1,2-b]pyridazin-3-sulfonamide

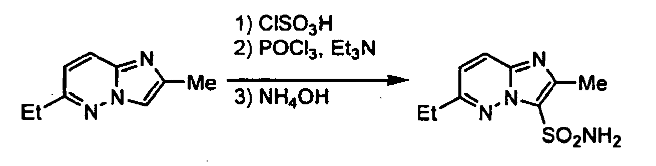

6-Ethyl-2-methylimidazo[1,2-b]pyridazine (2.70 g, 16.7 mmol) was dissolved in 1,2-dichloroethane (30 ml), and chlorosulfonic acid (1.27 g, 18.5 mmol) was added thereto under stirring at room temperature, and the mixture was stirred for 5 hours under reflux with heating. Then, the reaction solution was cooled to about 70°C, and triethylamine (2.38 g, 23.5 mmol) was added dropwise thereto over 1 minute. After dropping, the reaction solution was stirred for 20 minutes under reflux with heating. Thereafter, the reaction solution was cooled to about 70°C, and phosphorus oxychloride (3.86 g, 25.2 mmol) was added dropwise thereto over 1 minute. After dropping, the mixture was stirred for 2 hours under reflux with heating. The reaction solution was left to cool to about 50°C, and poured into 50 ml warm water (about 50°C). The reaction mixture was stirred for 5 minutes, and the organic layer was separated. The aqueous layer was extracted with chloroform (50 mlx2). The organic layers were combined, washed with water, dried over magnesium sulfate, and concentrated. The residues were dissolved in acetonitrile (40 ml), and 14 N ammonia water (7 ml) was added thereto under stirring at room temperature, and the mixture was stirred at room temperature for 2 hours. After the reaction was completed, the reaction solution was poured onto ice-cold water (150 ml) and adjusted to about pH 4 with concentrated hydrochloric acid, to form crystals which were then collected by filtration, washed with water and dried under reduced pressure. Thereafter, the crystals were purified by silica gel column chromatography (chloroform : acetone = 9 : 1 → 4 : 1). The title compound was obtained as white crystals. The yield was 1.8 g (44.7%).
mp 215.0-215.5°C
¹H NMR (DMSO-d₆, δ): 1.30 (3H,t,J=7.5 Hz), 2.57 (3H,s), 2. 93 (2H,q,J=7.5 Hz), 7.39 (1H,d,J=9.3 Hz), 7.47 (2H,brs), 8.08 (1H,d,J=9.3 Hz)
IR (Nujol, cm⁻¹): 3304, 3177, 3090, 1546, 1540, 1507, 1463, 1389, 1362, 1341, 1309, 1201, 1166, 1127, 1086, 1057, 959, 900, 9864, 824, 772, 686, 670, 652, 591, 525

### Example 3

### Synthesis of 2-chloro-6-n-propylimidazo[1,2-b]pyridazine

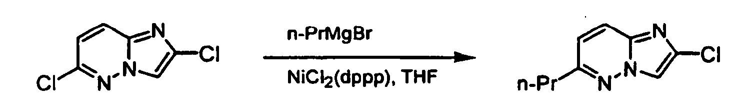

2,6-Dichloroimidazo[1,2-b]pyridazine (10.0 g, 53.2 mmol) and [1,3-bis(diphenylphosphino)propane]nickel(II) dichloride (0.43 g, 0.80 mmol) were added to tetrahydrofuran (80.0 ml) under a nitrogen stream, and a solution of n-propylmagnesium bromide in tetrahydrofuran (2 M, 31.9 ml, 63.8 mmol) was added dropwise thereto under ice-cooling over 60 minutes. The reaction mixture was stirred for 10 minutes under ice-cooling, warmed to room temperature, and stirred for 2 hours at room temperature. To the reaction mixture was added cold water (700 ml), and acidified with concentrated hydrochloric acid. Then the deposited solid was collected by filtration, and insoluble solid was washed with dilute hydrochloric acid, then with water. At the same time, the filtrate was extracted with ethyl acetate, the extracts were combined, and washed with dilute hydrochloric acid, saturated brine, saturated sodium hydrogen carbonate solution, and saturated brine, in that order. The resulting organic layer was dried over anhydrous magnesium sulfate, filtered, and concentrated. The concentrated residue and the solid collected by filtration were purified by silica gel column chromatography (ethyl acetate : hexane = 3 : 7) to give the title compound as white crystals. The yield was 9.21 g (88.5%).
mp: 73.9-80.0°C
¹H NMR(CDCl₃, δ): 1.01(3H, t, J=7.4 Hz), 1.78(2H, m), 2.79(2H, t, J=7.6 Hz), 6.96 (1H, d, J=9.3 Hz), 7.75(1H, d, J=9.3 Hz), 7.80(1H, s).
IR(Nujol, cm⁻¹): 3122, 1466, 1377, 1314, 1302.

### Example 4

### Synthesis of 2-chloro-6-n-propylimidazo[1,2-b]pyridazin-3-sulfonamide

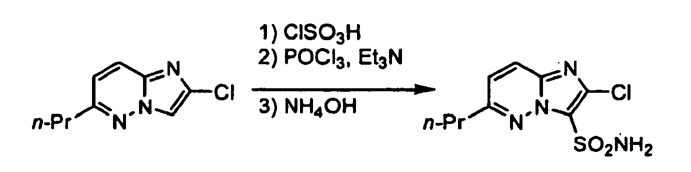

2-Chloro-6-n-propylimidazo[1,2-b]pyridazine (0.8 g, 4.1 mmol) and dichloroethane (10 ml) were introduced into a 200-ml eggplant type flask and stirred at room temperature, and chlorosulfonic acid (0.54 g, 4.5 mmol) was added thereto all at once, and the mixture was stirred for 4 hours under reflux with heating. The reaction solution was cooled to about 70°C, and triethylamine (0.5 g, 5 mmol) was added thereto all at once and stirred until the solid was dissolved, and phosphorus oxychloride (0.79 g, 5 mmol) was added thereto all at once, and the mixture was stirred for 2 hours under reflux with heating. After the reaction was completed, the reaction solution was left to cool, and water (50 ml) was added thereto and the organic phase was separated. The organic phase was washed with a saturated saline, dried over magnesium sulfate and concentrated. Acetonitrile (10 ml) and 28% ammonia water (4 ml) were added to the residue and stirred at room temperature for 2 hours. After the reaction was completed, water (100 ml) was added to the reaction solution, which was then adjusted to about pH 2 with dilute hydrochloric acid, and the formed crystals were collected by filtration, washed with water and chloroform, and dried under reduced pressure to give the title compound as pale brown crystals. The yield was 0.49 g (43.5%).
mp 174-5°C
¹H NMR (DMSO-d₆, δ): 0.96 (3H,t,J=7.4 Hz), 1.7-1.9 (2H,m), 2.8-3.0 (2H,m), 7.53 (1H,d,J=9.5 Hz), 7.82 (2H,brs), 8.19 (1H,d,J=9.4 Hz)
IR (Nujol, cm⁻¹): 3377, 3324, 3189, 1545, 1364, 1322, 1187, 1166, 821, 680, 597

### Example 5

### Synthesis of 6-n-butyl-2-chloroimidazo[1,2-b]pyridazine

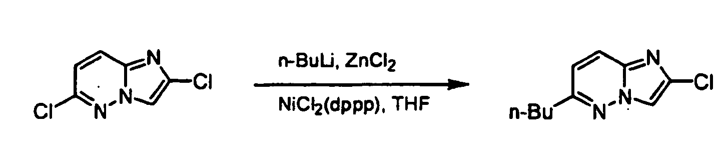

Zinc chloride (2.04 g, 15.0 mmol) was dried at 180°C for 2 hours under vacuum and then cooled to room temperature, and anhydrous tetrahydrofuran (20.0 mL) was added thereto. n-Butyl lithium (1.6 M, 9.0 mL, 14.4 mmol) was added dropwise thereto over about 30 minutes under ice-cooling and stirred for 30 minutes under ice-cooling, to prepare a solution of n-butylzinc chloride in tetrahydrofuran. Separately, a suspension of 2,6-dichloroimidazo[1,2-b]pyridazine (1.88 g, 10.0 mmol) and [1,3-bis(diphenylphosphino)propane]nickel(II) dichloride (0.16 g, 0.30 mmol) in anhydrous tetrahydrofuran (20.0 mL) was prepared under a nitrogen atmosphere, and the previously prepared solution of n-butylzinc chloride in tetrahydrofuran was added dropwise thereto over 30 minutes with maintaining at 3 to 6°C. The mixture was stirred for 15 minutes under ice-cooling and for 3 hours at room temperature, poured into a saturated saline and adjusted to pH 2 with dilute hydrochloric acid. The reaction solution was extracted twice with ethyl acetate, and the extracts were combined, dehydrated over anhydrous magnesium sulfate and concentrated under reduced pressure. The residues were purified by silica gel column chromatography (ethyl acetate : hexane = 1 : 4) to give the title compound as pale yellow crystals. The yield was 2.03 g (96.8%).
mp 61.0-63.0°C
¹H NMR (CDCl₃, δ): 0.96(3H, t, J=7.3 Hz),1.41(2H, tq, J=7.5, 7.3 Hz),1.73(2H, tt, J=7.8, 7.5 Hz), 2.81(2H, t, J=7.8 Hz), 6.96(1H, d, J=9.4 Hz), 7.74(1H, d, J=9.4 Hz), 7.79(1H, s). IR(Nujol, cm⁻¹): 3115, 3061, 1545, 1466, 1378, 1326, 1276, 817.

### Example 6

### Synthesis of 6-n-butyl-2-chloroimidazo[1,2-b]pyridazin-3-ylsulfonamide

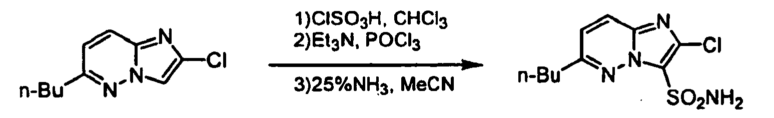

6-n-Butyl-2-chloroimidazo[1,2-b]pyridazine (1.00 g, 4.77 mmol) was dissolved in chloroform (10.0 mL), and chlorosulfonic acid (0.35 mL, 5.27 mmol) was added dropwise to the solution under stirring at room temperature. After the mixture was heated for 5 hours under reflux, it was confirmed by TLC that the starting material remained, so additional chlorosulfonic acid (0.35 mL, 5.27 mmol) was added thereto, and the mixture was heated for 4 hours under reflux. The resulting suspension was left to cool to room temperature, and triethylamine (2.50 mL, 17.9 mmol) and phosphorus oxychloride (2.00 mL, 21.5 mmol) were added thereto, and the mixture was heated again for 4 hours under reflux. The reaction solution was cooled to room temperature, poured into water and extracted 3 times with chloroform, and the extracts were combined, dehydrated over anhydrous magnesium sulfate and concentrated under reduced pressure to give 3.24 g dark red liquid. This liquid was dissolved in acetonitrile (10.0 mL) and added dropwise to a solution of 25% ammonia water (5.00 g, 73.5 mmol) in acetonitrile (15.0 mL) under ice-cooling. The mixture was stirred for 30 minutes under ice-cooling and for 1 hour at room temperature, and then the acetonitrile was distilled away under reduced pressure. The residues were adjusted to pH 2 with dilute hydrochloric acid and extracted twice with chloroform, and the chloroform layers were combined, dehydrated over anhydrous magnesium sulfate and concentrated under reduced pressure. The residues were purified by silica gel column chromatography (ethyl acetate : hexane = 1 : 1 → chloroform : ethanol = 20 : 1) to give the title compound as white crystals. The yield was 0.92 g (66.8%).
mp 165.5-166.5°C
¹H NMR(DMSO-d₆, δ): 0.93(3H, t, J=7.3 Hz), 1.37(2H, tq, J=7.5, 7.3 Hz), 1.72(2H, tt, J=7.9, 7.5 Hz), 2.93(2H, t, J=7.9 Hz), 7.53(1H, d, J=9.4 Hz), 7.80(2H, s), 8.18(1H, d, J=9.4 Hz).
IR(Nujol, cm⁻¹): 3412, 3360, 3287, 3197, 1546, 1464, 1376, 1321, 1172.

### Example 7

### Synthesis of N'-(2-chloro-6-cyclopropylimidazo[1,2-b]pyridazin-3-ylsulfonyl)-N,N-diisobutylformamidine

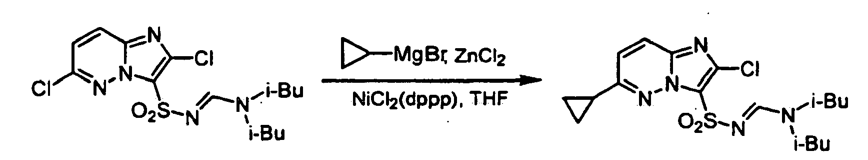

Magnesium metal powder (0.27 g, 11.1 mmol) was mixed with iodine (5 mg), heated with a dryer under a nitrogen atmosphere and cooled to room temperature, and anhydrous tetrahydrofuran (15.0 mL) was added thereto. Cyclopropyl bromide (1.33 g, 1.10 mmol), while keeping at 28 to 33°C, was added dropwise to the mixture under stirring at room temperature, and then the mixture was stirred at room temperature for 30 minutes to prepare a pale yellowish gray solution of cyclopropylmagnesium bromide in tetrahydrofuran. Separately, zinc chloride (1.50 g, 11.0 mmol) dried at 180°C for 4 hours under vacuum was dissolved in anhydrous tetrahydrofuran (10.0 mL) under a nitrogen atmosphere and then the previously prepared solution of cyclopropylmagnesium bromide in tetrahydrofuran was added dropwise thereto with keeping at 0°C or less with an ice-sodium chloride bath. The mixture was stirred at about - 10°C for 15 minutes, and [1,3-bis(diphenylphosphino)propane]nickel(II) dichloride (0.27 g, 0.50 mmol) was added as powder to the resulting suspension, and then a solution of N'-(2,6-dichloroimidazo[1,2-b]pyridazin-3-ylsulfonyl)-N,N-diisobutylformamidine (2.03 g, 5.00 mmol) dissolved in anhydrous tetrahydrofuran (10.0 mL) was added dropwise thereto. The mixture was stirred at - 10°C for 2 hours, then at room temperature for 16 hours, poured into a saturated saline, adjusted to pH 2 with dilute hydrochloric acid, and extracted 4 times with chloroform. The extracts were combined, dehydrated over anhydrous magnesium sulfate, concentrated under reduced pressure, and the residues were purified by silica gel column chromatography (ethyl acetate : hexane = 1 : 1), whereby 0.64 g (31.5%) of the starting material N'-(2,6-dichloroimidazo[1,2-b]pyridazin-3-ylsulfonyl)-N,N-diisobutylformamidine was recovered and simultaneously the title compound was obtained as pale yellow crystals. The yield was 0.94 g (45.7%).
mp 154.0-160.0°C
¹H NMR(CDCl₃, δ): 0.74(6H, d, J=6.7 Hz), 0.95(6H, d, J=6.7 Hz), 1.00-1.10(2H, m), 1.10-1.25(2H, m), 1.85-2.10(2H, m), 2.10-2.20(1H, m), 3.19(2H, d, J=7.5 Hz), 3.28(2H, d, J=7.5 Hz), 6.98(1H, d, J=9.4 Hz), 7.78(1H, d, J=9.4 Hz), 8.45(1H, s).
IR(Nujol) ν (cm⁻¹): 1613, 1464, 1334, 1318, 1143, 909, 859, 661.

### Example 8

### Synthesis of 2-chloro-6-cyclopropylimidazo[1,2-b]pyridazin-3-ylsulfonamide

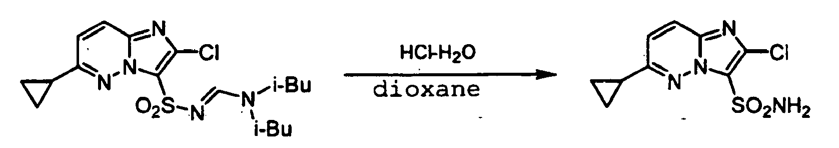

N'-(2-Chloro-6-cyclopropylimidazo[1,2-b]pyridazin-3-ylsulfonyl)-N,N-diisobutylformamidine (0.93 g, 2.26 mmol) was dissolved in dioxane (9.00 mL), and 36% concentrated hydrochloric acid (9.0 mL, 107 mmol) was added dropwise to the solution under stirring at 100°C. The mixture was stirred for 15 hours at 100 to 105°C, then left to cool to room temperature and concentrated under reduced pressure until crystals occurred. Water (30.0 mL) was poured into the residues, and the crystals were completely precipitated, then filtered, washed with water and washed with methanol, to give the title compound as white crystals. The yield was 0.31 g (50.4%).
mp 194.0-196.0°C
NMR(DMSO-d₆, δ): 1.10-1.25(4H, m), 2.30-2.45(1H, m), 7.36(1H, d, J=9.4 Hz), 7.78(2H, brs), 8.12(1H, d, J=9.4Hz). IR(Nujol, cm⁻¹): 3348, 3247, 1553, 1468, 1455, 1358, 1316, 1170, 908, 825, 662.

### Example 9

### Synthesis of N'-(2-chloro-6-ethenylimidazo[1,2-b]pyridazin-3-ylsulfonyl)-N,N-diisobutylformamidine

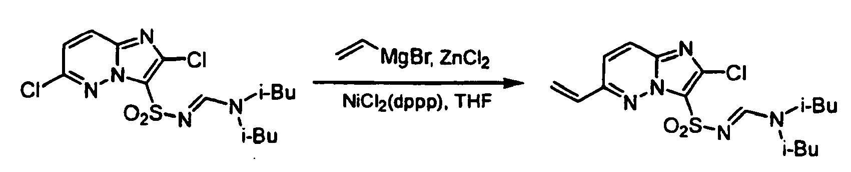

The title compound was obtained as pale yellow crystals by the same reaction as in Example 7 except that a solution of commercially available vinyl magnesium bromide in tetrahydrofuran was used in place of the solution of cyclopropylmagnesium bromide in tetrahydrofuran, and [1,3-bis(diphenylphosphino)propane]nickel(II) dichloride was used in an amount of 3 mol-% relative to the starting material N'-(2,6-dichloroimidazo[1,2-b]pyridazin-3-ylsulfonyl)-N,N-diisobutylformamidine. The yield was 80.4%. mp 194.0-198.0°C
¹H NMR(CDCl₃, δ): 0.71(6H, d, J=6.7 Hz), 0.94(6H, d, J=6.6 Hz), 1.85-2.10(2H, m), 3.17(2H, d, J=7.5 Hz), 3.26(2H, d, J=7.7 Hz), 5.77(1H, d, J=11.1 Hz), 6.16(1H, d, J=17.8 Hz), 6.82(1H, dd, J=17.8, 11.1 Hz), 7.46(1H, d, J=9.5 Hz), 7.89(1H, d, J=9.5 Hz), 8.50(1H, s).
IR(Nujol, cm⁻¹): 1614, 1456, 1350, 1319, 1145, 913, 859, 664, 612.

### Example 10

### Synthesis of 2-chloro-6-ethenylimidazo[1,2-b]pyridazin-3-ylsulfonamide

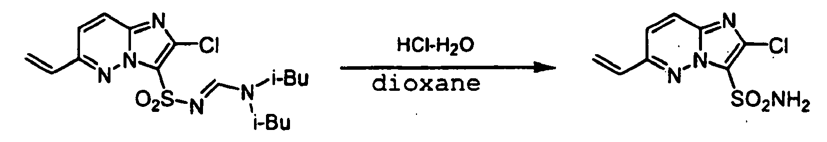

The reaction was carried out in the same manner as in Example 8 except that N'-(2-chloro-6-ethenylimidazo[1,2-b]pyridazin-3-ylsulfonyl)-N,N-diisobutylformamidine was used in place of N'-(2-chloro-6-cyclopropylimidazo[1,2-b]pyridazin-3-ylsulfonyl)-N,N-diisobutylformamidine. The resulting crystals were purified by silica gel column chromatography (chloroform : methanol = 10 : 1) to give the title compound as white crystals. The yield was 42.1%.
mp 229.0-233.0°C
¹H NMR(DMSO-d₆, δ): 5.87(1H, d, J=11.2 Hz), 6.50(1H, d, J=17.9 Hz), 6.86(1H, dd, J=17.9, 11.2 Hz), 7.89(2H, s), 7.96(1H, d, J=9.6 Hz), 8.26(1H, d, J=9.6 Hz).
IR(Nujol, cm⁻¹): 3316, 3183, 1466, 1368, 1321, 1167.

### Example 11

### Synthesis of N'-(2-chloro-6-(1-propenyl)imidazo[1,2-b]pyridazin-3-ylsulfonyl)-N,N-diisobutylformamidine

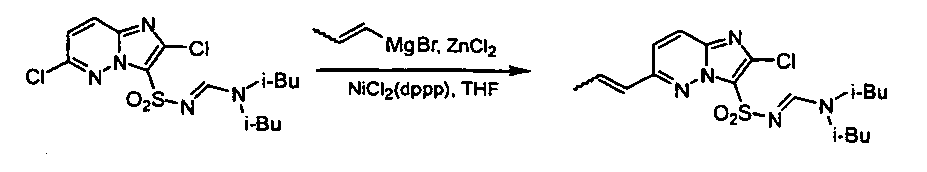

The title compound was obtained as a mixture of E and Z (E : Z = 5 : 3) in the form of pale yellow crystals by the same reaction as in Example 7 except that a solution of commercially available 1-propenylmagnesium bromide in tetrahydrofuran was used in place of the solution of cyclopropylmagnesium bromide in tetrahydrofuran, and [1,3-bis(diphenylphosphino)propane]nickel(II) dichloride was used in an amount of 3 mol-% relative to the starting material N'-(2,6-dichloroimidazo[1,2-b]pyridazin-3-ylsulfonyl)-N,N-diisobutylformamidine. The yield was 100%. mp: not be measured because of a mixture of E and Z.
¹H NMR(CDCl₃, δ):[E isomer] 0.72(6H, d, J=6.6 Hz), 0.94(6H, d, J=6.6 Hz), 1.85-2.10(2H, m), 2.00(3H, dd, J=6.9, 1.5 Hz), 3.17(2H, d, J=7.6 Hz), 3.26(2H, d, J=7.7 Hz), 6.51(1H, dq, J=16.0, 1.5 Hz), 6.71(1H, dq, J=16.0, 6.9 Hz), 7.35(1H, d, J=9.5 Hz), 7.82(1H, d, J=9.5 Hz), 8.50(1H, s).
¹H NMR(CDCl₃, δ):[Z isomer] 0.72(6H, d, J=6.6 Hz), 0.92(6H, d, J=6.6 Hz), 1.85-2.10(2H, m), 2.21(3H, dd, J=7.3, 1.8Hz), 3.12(2H, d, J=7.5 Hz), 3.25(2H, d, J=7.7 Hz), 6.23(1H, dq, J=11.9, 7.3 Hz), 6.40(1H, dq, J=11.9, 1.8 Hz), 7.19(1H, d, J=9.5 Hz), 7.85(1H, d, J=9.5 Hz), 8.43(1H s).
IR(Nujol, cm⁻¹): 1609, 1456, 1351, 1319, 1144, 911.

### Example 12

### Synthesis of (E)-2-chloro-6-(1-propenyl)imidazo[1,2-b]pyridazin-3-ylsulfonamide

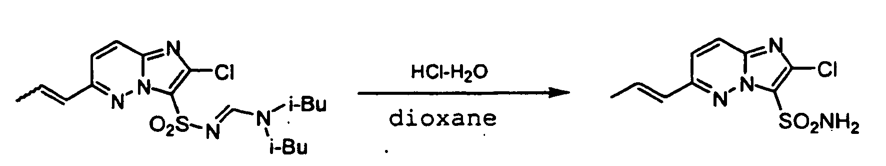

The reaction was carried out in the same manner as in Example 8 except that N'-(2-chloro-6-(1-propenyl)imidazo[1,2-b]pyridazin-3-ylsulfonyl)-N,N-diisobutylformamidine as a mixture of E and Z was used in place of N'-(2-chloro-6-cyclopropylimidazo[1,2-b]pyridazin-3-ylsulfonyl)-N,N-diisobutylformamidine. The resulting crystals were purified by silica gel column chromatography (chloroform : methanol = 20 : 1) to give the title compound as white crystals. The yield was 70.1%.
mp 225.0-229.0°C
¹H NMR(DMSO-d₆, δ): 1.98(3H, dd, J=6.8, 1.7 Hz), 6.71(1H, dq, J=16.0, 1.7 Hz), 7.01(1H, dq, J=16.0, 6.8 Hz), 7.83(2H, s), 7.84(1H, d, J=9.5 Hz), 8.19(1H, d, J=9.6 Hz).
IR(Nujol, cm⁻¹): 3323, 3179, 1662, 1550, 1466, 1360, 1325, 1173.

### Example 13

### Synthesis of 2-chloro-6-isobutylimidazo[1,2-b]pyridazine

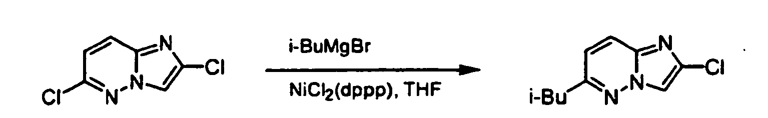

The reaction was carried out in the same manner as in Example 3 except that a solution of isobutylmagnesium bromide in tetrahydrofuran was used in place of the solution of n-propylmagnesium bromide in tetrahydrofuran. The resulting crude product was purified by silica gel column chromatography (ethyl acetate : hexane = 1 : 4) to give the title compound as pale yellow crystals. The yield was 1.27 g (60.6%).
mp 71.0-72.5°C
¹H NMR(CDCl₃, δ): 0.98(6H, d, J=6.6 Hz), 2.09(1H, m), 2.68(2H, d, J=7.3 Hz), 6.94(1H, d, J=9.3 Hz), 7.75(1H, d, J=9.3 Hz), 7.81(1H,s).
IR(Nujol, cm⁻¹): 3126, 3059, 1545, 1466, 1369, 1331, 1320, 1279, 803.

### Example 14

### Synthesis of 2-chloro-6-isobutylimidazo[1,2-b]pyridazin-3-ylsulfonamide

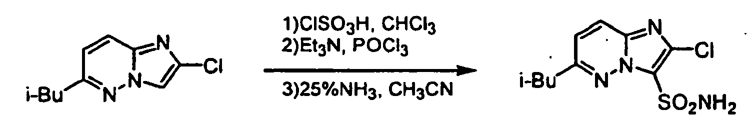

The reaction was carried out in the same manner as in Example 6 except that 2-chloro-6-isobutylimidazo[1,2-b]pyridazine was used in place of 2-chloro-6-n-butylimidazo[1,2-b]pyridazine. The resulting reaction mixture was purified by silica gel column chromatography (ethyl acetate : hexane = 1 : 1) to give the title compound as white crystals. The yield was 1.12 g (64.0%).
mp 168.0-169.5°C
¹H NMR(DMSO-d₆, δ): 0.93(6H, d, J=6.6 Hz), 2.14(1H, m), 2.82(2H, d, J=7.4 Hz), 7.51(1H, d, J=9.4 Hz), 7.80(2H, s), 8.19(1H, d, J=9.4Hz).
IR(Nujol, cm⁻¹): 3316, 3180, 3117, 1548, 1469, 1362, 1336, 1321, 1200, 1173, 849, 678.

### Example 15

### Synthesis of 2-chloro-6-ethylimidazo[1,2-b]pyridazine

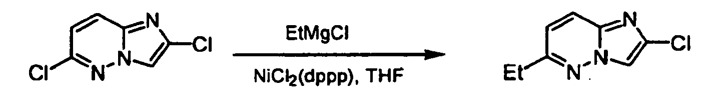

The title compound was obtained as pale yellow crystals by the same reaction as in Example 3 except that a solution of ethylmagnesium chloride in tetrahydrofuran was used in place of the solution of n-propylmagnesium bromide in tetrahydrofuran. The yield was 66.2%.
¹H NMR(CDCl₃, δ): 1.35(3H, t, J=7.6 Hz), 2.85(2H, q, J=7.6 Hz),6.97(1H, d, J=9.3 Hz), 7.75(1H, d, J=9.3 Hz), 7.80(1H, s).
IR(Nujol, cm⁻¹): 3121, 3058, 1544, 1471, 1318, 1280, 1262, 1189, 1142, 1121, 1059, 983, 953, 822.

### Example 16

### Synthesis of 2-chloro-6-ethylimidazo[1,2-b]pyridazin-3-ylsulfonamide

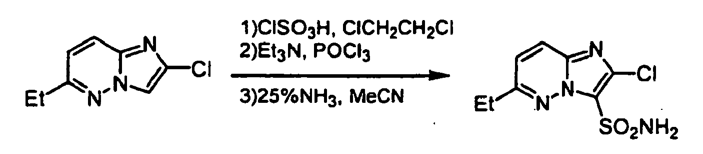

The title compound was obtained as pale brown crystals by the same reaction as in Example 4 except that 2-chloro-6-ethylimidazo[1,2-b]pyridazine was used in place of 2-chloro-6-n-propylimidazo[1,2-b]pyridazine. The yield was 74.1%.
mp 204-205°C
¹H NMR(DMSO-d₆, δ): 1.31(3H, t, J=7.6 Hz), 2.95(2H, q, J=7.6 Hz), 7.54(1H, d, J=9.4 Hz), 7.82(2H, brs), 8.19(1H, d, J=9.4 Hz).
IR(Nujol, cm⁻¹): 3317, 3211, 1365, 1356, 1325, 1172, 829, 668.

### Example 17

### Synthesis of 2-methyl-6-n-propylimidazo[1,2-b]pyridazine

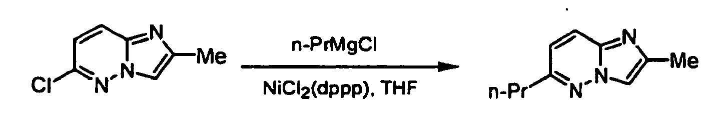

The title compound was obtained as pale reddish oil by the same reaction as in Example 1 except that a solution of n-propylmagnesium chloride in ether was used in place of the solution of ethylmagnesium bromide in ether, and as the solvent, a tetrahydrofuran solvent was used in place of the mixed solvent of ether and tetrahydrofuran. The yield was 19.1%.
¹H NMR(CDCl₃, δ): 1.00(3H, t, J=7.4 Hz), 1.7-1.9(2H, m), 2.48(3H, d, J=0.7 Hz), 2.77(2H, t, J=7.5 Hz), 6.85(1H, d, J=9.2 Hz), 7.66(1H, d, J=0.7 Hz), 7.72(1H, d, J=9.2 Hz). IR(Nujol, cm⁻¹): 2961, 1541, 1464, 1326, 1296, 1153, 1124, 989, 816, 726.

### Example 18

### Synthesis of 2-methyl-6-n-propylimidazo[1,2-b]pyridazin-3-ylsulfonamide

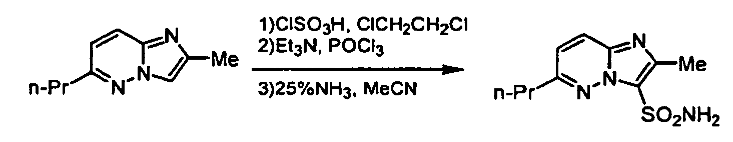

The title compound was obtained as pale brown crystals by the same reaction as in Example 4 except that 2-methyl-6-n-propylimidazo[1,2-b]pyridazine was used in place of 2-chloro-6-n-propylimidazo[1,2-b]pyridazine. The yield was 14.6%.
mp 178-179°C(dec.)
¹H NMR(DMSO-d₆, δ): 0.96(3H, t, J=7.3 Hz), 1.7-1.9(2H, m), 2.56(3H, s), 2.8-2.9(2H, m), 7.39(1H, d, J=9.3 Hz), 7.46(2H, brs), 8.08(1H, d, J=9.3 Hz).
IR(Nujol, cm⁻¹): 3384, 3327, 1543, 1508, 1420, 1348, 1327, 1309, 1162, 827.

### Example 19

### Synthesis of N,N-dimethyl-N'-(6-n-propyl-2-trifluoromethylimidazo[1,2-b]pyridazin-3-ylsulfonyl)formamidine

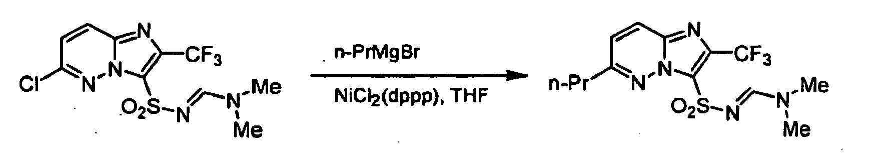

To a suspension of N'-(6-chloro-2-trifluoromethylimidazo[1,2-b]pyridazin-3-ylsulfonyl)-N,N-dimethylformamidine (1.00 g, 2.81 mmol) and [1,3-bis(diphenylphosphino)propane]nickel(II) dichloride (0.076 g, 0.14 mmol) in tetrahydrofuran (8.0 ml) was added dropwise a solution of n-propylzinc bromide in tetrahydrofuran (0.5 M, 8.43 ml, 4.22mmol) with stirring under ice-cooling and nitrogen stream. The reaction mixture was stirred for 30 minutes under ice-cooling and 4.5 hours at room temperature, then poured into cold water and made acid with dilute hydrochloric acid. The precipitated solid was collected by filtration, washed with dilute hydrochloric acid, then water, and purified by silica gel column chromatography (ethyl acetate : chloroform = 2 : 5) to give the title compound as white crystals. The yield was 0.62 g (60.7%).
mp 219.3-220.4°C
¹H NMR(DMSO-d₆, δ): 0.95(3H, t, J=7.3 Hz), 1.71(2H, m), 2.88(2H, t, J=7.7 Hz), 2.92(3H, s), 3.28(3H, s), 7.59(1H, d, J=9.5 Hz), 8.33(1H, d, J=9.5 Hz), 8.54(1H, s).
IR(Nujol, cm⁻¹): 1635, 1334, 1318, 1169, 1153, 920, 619.

### Example 20

### Synthesis of 6-n-propyl-2-trifluoromethylimidazo[1,2-b]pyridazin-3-ylsulfonamide

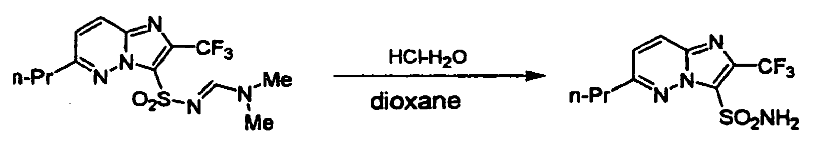

N,N-dimethyl-N'-(6-n-propyl-2-trifluoromethylimidazo[1,2-b]pyridazin-3-ylsulfonyl)formamidine (0.30 g, 0.83 mmol) was dissolved in dioxane (10.0 ml), and to the solution was added concentrated hydrochloric acid (5.0 ml) and stirred at 60°C for 2 hours, 80°C for 2 hours and 90°C for 2 hours. The reaction mixture was concentrated under reduced pressure. To the residues was added water, and adjusted pH to 3 with aqueous 1N sodium hydroxide solution. The precipitated solid was collected by filtration, and washed with water to give the title compound as white crystals. The yield was 0.24 g (94.3%).
mp 151.0-151.7°C
¹H NMR(DMSO-d₆, δ): 0.97(3H, t, J=7.3 Hz), 1.78(2H, m), 2.96(2H, t, J=7.7 Hz), 7.62(1H, d, J=9.5 Hz), 7.97(2H, brs), 8.36(1H, d, J=9.5 Hz).
IR(Nujol, cm⁻¹): 3356, 1550, 1465, 1373, 1362, 1322, 1199, 1179, 1151, 608.

### Example 21

### Synthesis of 2-chloro-6-n-propylimidazo[1,2-b]pyridazine

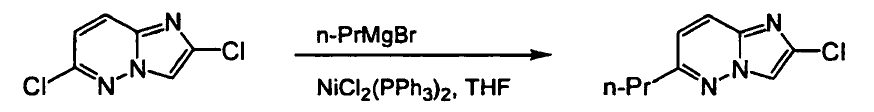

2,6-Dichloroimidazo[1,2-b]pyridazine (0.50 g, 2.66 mmol) and bis(triphenylphosphine)nickel(II) dichloride (0.17 g, 0.27 mmol) were added to tetrahydrofuran (5.0 ml) under a nitrogen stream, and a solution of n-propylmagnesium bromide in tetrahydrofuran (2 M, 1.99 ml, 3.99 mmol) was added dropwise over 10 minutes to the mixture under ice-cooling. The mixture was stirred for 10 minutes under ice-cooling, and the reaction mixture was warmed to room temperature and stirred for 4 hours at room temperature. Cold water (50 ml) was added to the reaction mixture which was then acidified with dilute hydrochloric acid, and extracted with ethyl acetate. The extracts were washed with dilute hydrochloric acid, a saturated saline, an aqueous saturated sodium bicarbonate solution and a saturated saline in this order. The resulting organic layer was dried over anhydrous magnesium sulfate, filtrated and concentrated. The concentrated residue was purified by silica gel column chromatography (ethyl acetate : hexane = 3 : 7) to give the title compound as white crystals. The yield was 0.21 g (40.4%).

### Example 22

### Synthesis of 2-chloro-6-n-propylimidazo[1,2-b]pyridazine

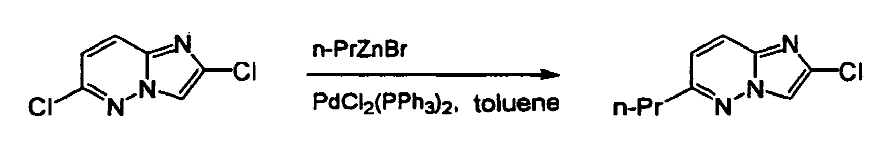

Under a nitrogen stream, a solution of n-propylzinc bromide in tetrahydrofuran (0.5 M, 7.98 ml, 3.99 mmol) was diluted with toluene (5.0 ml), and 2,6-dichloroimidazo[1,2-b]pyridazine (0.50 g, 2.66 mmol) and bis(triphenylphosphine)palladium(II) dichloride (0.19 g, 0.27 mmol) were added thereto, and the reaction mixture was stirred for 2 hours at 80°C. After cooling, to the reaction mixture was added cold water (50 ml), acidified with dilute hydrochloric acid, extracted with ethyl acetate, and the extracts were washed with dilute hydrochloric acid, a saturated saline, an aqueous saturated sodium bicarbonate solution and a saturated saline in this order. The resulting organic layer was dried over anhydrous magnesium sulfate, filtrated and concentrated. The concentrated residue was purified by silica gel column chromatography (ethyl acetate : hexane = 3 : 7) to give the title compound as white crystals. The yield was 0.31 g (59.6%).

### Example 23

### Synthesis of 2-chloro-6-n-propylimidazo[1,2-b]pyridazine

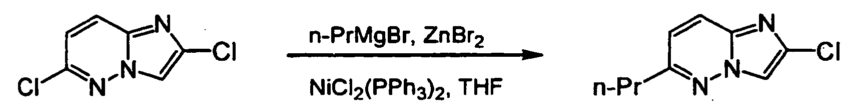

Under a nitrogen stream, 2,6-dichloroimidazo[1,2-b]pyridazine (1.00 g, 5.32 mmol), bis(triphenylphosphine)nickel(II) dichloride (0.10 g, 0.16 mmol) and zinc bromide (0.04 g, 0.16 mmol) were added to tetrahydrofuran (8.0 ml), and a solution of n-propylmagnesium bromide in tetrahydrofuran (2 M, 3.99 ml, 7.98 mmol) was added dropwise over 10 minutes to the mixture under ice-cooling. The reaction mixture was stirred for 10 minutes under ice-cooling, and warmed to room temperature and stirred for 4 hours at room temperature. Cold water (100 ml) was added to the reaction mixture which was then acidified with dilute hydrochloric acid, and extracted with ethyl acetate. The extracts were washed with dilute hydrochloric acid, a saturated saline, an aqueous saturated sodium bicarbonate solution and a saturated saline in this order. The resulting organic layer was dried over anhydrous magnesium sulfate, filtrated and concentrated. The concentrated residue was purified by silica gel column chromatography (ethyl acetate : hexane = 3 : 7) to give the title compound as pale yellow crystals. The yield was 0.76 g (73.1%).

### Example 24

### Synthesis of 2-chloro-6-n-propylimidazo[1,2-b]pyridazine

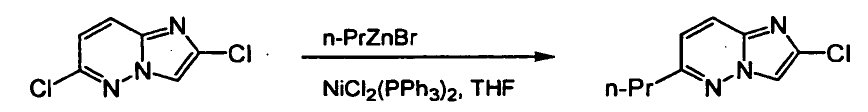

Under a nitrogen stream, 2,6-dichloroimidazo[1,2-b]pyridazine (0.50 g, 2.66 mmol) and bis(triphenylphosphine)nickel(II) dichloride (0.17 g, 0.27 mmol) were added to tetrahydrofuran (5.0 ml), and a solution of n-propylzinc bromide in tetrahydrofuran (0.5 M, 7.98 ml, 3.99 mmol) was added dropwise over 10 minutes to the mixture under ice-cooling. The reaction mixture was stirred for 10 minutes under ice-cooling, and warmed to room temperature and stirred for 2 hours at room temperature. Cold water (50 ml) was added to the reaction mixture which was then acidified with dilute hydrochloric acid, and extracted with ethyl acetate. The extracts were washed with dilute hydrochloric acid, a saturated saline, an aqueous saturated sodium bicarbonate solution and a saturated saline in this order. The resulting organic layer was dried over anhydrous magnesium sulfate, filtrated and concentrated. The concentrated residue was purified by silica gel column chromatography (ethyl acetate : hexane = 3 : 7) to give the title compound as white crystals. The yield was 0.43 g (82.7%).

### Example 25

### Synthesis of 2-chloro-6-n-propylimidazo[1,2-b]pyridazine

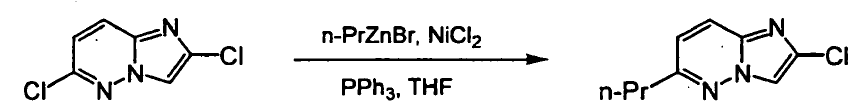

Under a nitrogen stream, anhydrous nickel(II) chloride (0.036 g, 0.27 mmol) and triphenylphosphine (0.15 g, 0.53 mmol) were added to tetrahydrofuran (5.0 ml), and stirred for 1 hour at room temperature. To this mixed solution was added 2,6-dichloroimidazo[1,2-b]pyridazine (0.50 g, 2.66 mmol), and a solution of n-propylzinc bromide in tetrahydrofuran (0.5 M, 7.98 ml, 3.99 mmol) was added dropwise thereto over 10 minutes under ice-cooling. The reaction mixture was stirred for 10 minutes under ice-cooling, and warmed to room temperature and stirred for 2 hours at room temperature. Cold water (50 ml) was added to the reaction mixture which was then acidified with dilute hydrochloric acid, and extracted with ethyl acetate. The extracts were washed with dilute hydrochloric acid, a saturated saline, an aqueous saturated sodium bicarbonate solution and a saturated saline in this order. The resulting organic layer was dried over anhydrous magnesium sulfate, filtrated and concentrated. The concentrated residue was purified by silica gel column chromatography (ethyl acetate : hexane = 3 : 7) to give the title compound as white crystals. The yield was 0.46 g (88.5%).

### Example 26

### Synthesis of 2-chloro-6-n-propylimidazo[1,2-b]pyridazine

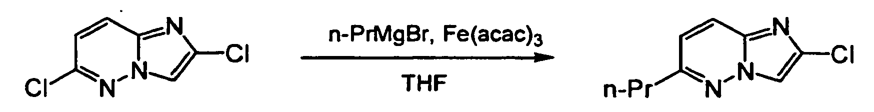

Under a nitrogen stream, 2,6-dichloroimidazo[1,2-b]pyridazine (0.50 g, 2.66 mmol) and iron(III) acetylacetonate (0.094 g, 0.27 mmol) were added to tetrahydrofuran (5.0 ml), and a solution of n-propylmagnesium bromide in tetrahydrofuran (2 M, 1.99 ml, 3.99 mmol) was added dropwise over 13 minutes to the mixture with stirring at 0 to 10°C. After stirring for 10 minutes under ice-cooling, the reaction mixture was warmed to room temperature and stirred for 6 hours at room temperature. The reaction mixture was poured onto ice-cold water, acidified with concentrated hydrochloric acid, and extracted with ethyl acetate. The extracts were washed with dilute hydrochloric acid, an aqueous saturated sodium bicarbonate solution and a saturated saline. The resulting organic layer was dried over anhydrous magnesium sulfate, filtrated and concentrated. The residue was purified by silica gel column chromatography (ethyl acetate : hexane = 3 : 7) to give the title compound as pale yellow crystals. The yield was 0.28 g (53.8%).

### Reference Example 1

### Synthesis of N'-(2,6-dichloroimidazo[1,2-b]pyridazin-3-ylsulfonyl)-N,N-dimethylformamidine

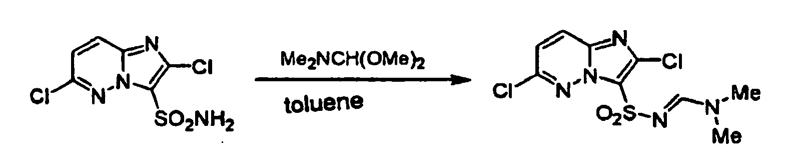

2,6-Dichloroimidazo[1,2-b]pyridazin-3-ylsulfonamide (2.00 g, 6.22 mmol) and N,N-Dimethylformamide dimethyl acetal (1.80 ml, 13.5 mmol) were heated under reflux for 4 hours in toluene (20.0 ml). The resulting reaction solution was left to cool to room temperature, and concentrated to dryness under reduced pressure to give the title compound as pale yellow crystals. The yield was 2.36 g (100%).
¹H NMR(DMSO-d₆, δ): 2.94(3H, s), 3.26(3H, s), 7.71(1H, d, J=9.5 Hz), 8.34(1H, d, J=9.5 Hz), 8.43(1H, s).

### Reference Example 2

### Synthesis of N'-(2,6-dichloroimidazo[1,2-b]pyridazin-3-ylsulfonyl)-N,N-diisobutylformamidine

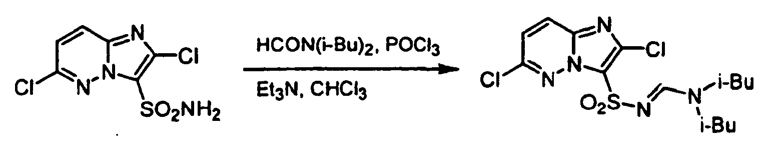

N,N-Diisobutylformamide (5.44 g, 34.5 mmol) was dissolved in chloroform (25.0 mL), and under cooling in an ice-sodium chloride bath, phosphorus oxychloride (3.22 mL, 34.5 mmol) was added dropwise thereto at -2°C or less. After stirring at -2°C or less for 30 minutes, to the mixture was added 2,6-dichloroimidazo[1,2-b]pyridazin-3-ylsulfonamide (6.15 g, 23.0 mmol). The mixture was stirred at -10°C for 10 minutes, and triethylamine (19.3 mL, 138 mmol) was added dropwise over 20 minutes to the solution at 5°C or less. The mixture was stirred for 1 hour at 0°C or less and for 1 hour at room temperature, then poured into an aqueous saturated sodium bicarbonate and extracted 5 times with chloroform. The extracts were combined, dehydrated over anhydrous magnesium sulfate, and concentrated under reduced pressure. The residues were purified by silica gel column chromatography (ethyl acetate : hexane = 1 : 1) to give the title compound as pale yellow crystals. The yield was 5.58 g (59.6%).
mp 151.0-154.0°C
¹H NMR(CDCl₃, δ): 0.76(6H, d, J=6.7 Hz), 0.97 (6H, d, J=6.7 Hz), 1.90-2.10(2H, m), 3.23(2H, d, J=7.6 Hz), 3.28(2H, d, J=7.7 Hz), 7.26(1H, d, J=9.5 Hz), 7.90(1H, d, J=9.5 Hz), 8.51(1H, s).
IR(Nujol, cm⁻¹): 1615, 1456, 1324, 1311, 1146, 910, 858, 654.

### Reference Example 3

### Synthesis of N'-(6-chloro-2-trifluoromethylimidazo[1,2-b]pyridazin-3-ylsulfonyl)-N,N-dimethylformamidine

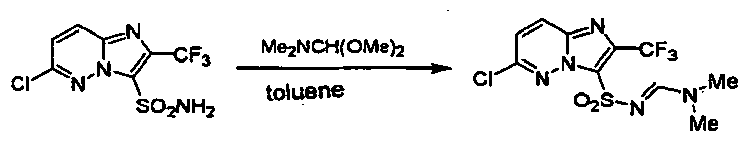

6-Chloro-2-trifluoromethylimidazo[1,2-b]pyridazin-3-ylsulfonamide (1.70 g, 5.65 mmol) was suspended in toluene (10.0 ml), and N,N-dimethylformamide dimethyl acetal (90%, 1.84 ml, 12.4 mmol) was added thereto and stirred for 3.5 hours under reflux. The reaction mixture was concentrated under reduced pressure. To the residue concentrated was added diisopropyl ether, and the crystals were collected by filtration to give the title compound as brown crystals. The yield was 1.96 g (97.4%).
mp 203.7-205.0°C
¹H NMR(CDCl₃, δ): 2.95(3H, s), 3.29(3H, s), 7.79(1H, d, J=9.6 Hz), 8.47(1H, s), 8.52(1H, d, J=9.6 Hz).
IR(Nujol, cm⁻¹): 1636, 1526, 1456, 1321, 1201, 1159, 1130, 1115, 922, 816, 628, 616.

### Industrial Applicability

According to the present invention, it becomes possible to produce easily and inexpensively an imidazo[1,2-b]pyridazin-3-ylsulfonamide derivative which has a substituent bonded to the 6-position carbon atom, which used to be difficult to produce by a conventional process for production, and using the same, sulfonylurea herbicides become possible to manufacture on a massive scale.

## Claims

1. A process for producing a compound represented by the formula (II): 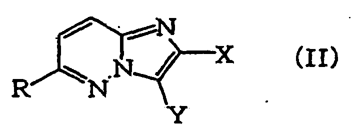
wherein X represents a chlorine atom,
Y represents a hydrogen atom. or SO₂N=CH-NR¹R² (wherein R¹ and R² represent each a C₁-C₆ alkyl group, or R¹ and R² may be combined together with the adjacent nitrogen atom to form a heterocyclic ring), and R represents a C₁₋₆ alkyl group, C₃₋₇ cycloalkyl group which may be substituted with C₁₋₆ alkyl, C₂₋₆ alkenyl group or C₂₋₆ alkynyl group, which comprises reacting an imidazo[1,2-b]pyridazine compound represented by the formula (I): 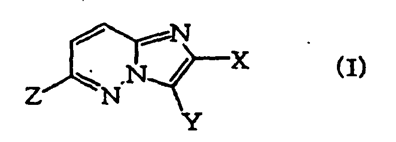
wherein X and Y are as defined above, and Z represents a chlorine atom with one or more compounds selected from the organometallic compounds represented by the formula: 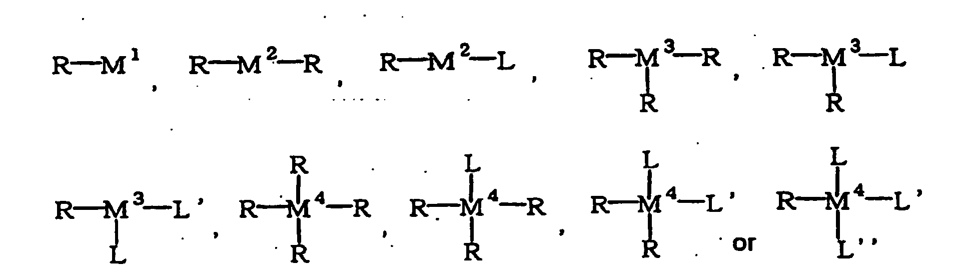
wherein R is as defined above, and M¹ represents an univalent metal, M² represents a divalent metal, M³ represents a trivalent metal and M⁴ represents a tetravalent metal, and L, L' and L" are the same or different and represent an anion, under the presence of transition metal catalyst.

2. The process according to claim 1, wherein the metal of the transition metal catalyst is palladium, nickel or iron.

3. The process according to claim 1, wherein the metal of the transition metal catalyst is nickel.

4. The process according to claim 1, wherein the metal of the organometallic compound is magnesium or zinc.

5. The process according to claim 1, wherein R is a C₁₋₆ alkyl group or C₃₋₇ cycloalkyl group which may be substituted with C₁₋₆ alkyl.

6. The process according to claim 1, wherein Y is a hydrogen atom and R is a C₁₋₆ alkyl group.

7. The process according to claim 3, wherein the metal of the organometallic compound is magnesium or zinc.

8. The process according to claim 7, wherein the organometallic compound is a C₁₋₆ alkylmagnesium halide or a C₁₋₆ alkylzinc halide.

9. The process according to claim 8, wherein the organometallic compound is a propylmagnesium halide or propylzinc halide and the nickel catalyst is [1,3-bis(diphenylphosphino)propane]nickel(II) dichloride or bis(triphenylphosphine)nickel(II) dichloride.

10. A process for producing a sulfonamide compound represented by the formula (III): 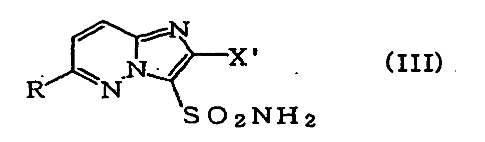 wherein X' represents a halogen atom or an optionally halogenated C₁₋₆ alkyl group and R represents a C₁₋₆ alkyl group, C₃₋₇ cycloalkyl group which may be substituted with C₁₋₆ alkyl, C₂₋₆ alkenyl group or C₂₋₆ alkynyl group, which comprises sulfonating with chlorosulfonic acid a compound represented by the formula (IIa) : 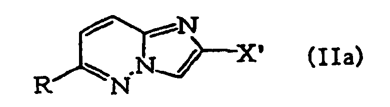 wherein X' and R are as defined above, which is obtained by reacting an imidazo[1,2-b]pyridazine compound represented by the formula (Ia): 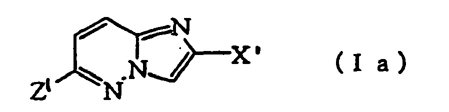 wherein X' is as defined above, and Z' represents a halogen atom or OSO₂R³ (wherein R³ represents an optionally fluorinated C₁₋₆ alkyl group or phenyl group which may be substituted with C₁₋₆ alkyl), with one or more compounds selected from the organometallic compounds represented by the formula: 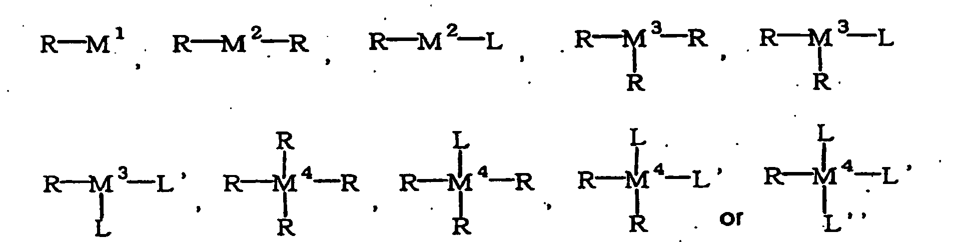 wherein R is as defined above, and M¹ represents an univalent metal, M² represents a divalent metal, M³ represents a trivalent metal and M⁴ represents a tetravalent metal, and L, L' and L" are the same or different and represent an anion, under the presence of transition metal catalyst, followed by converting to a sulfonyl chloride with phosphorus oxychloride, then reacting with ammonia.

11. A process for producing a sulfonamide compound represented by the formula (III): 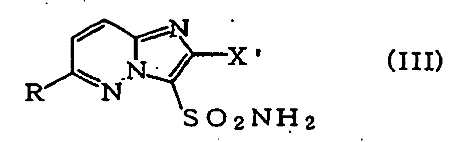 wherein X' represents a halogen atom or an optionally halogenated C₁₋₆ alkyl group and R represents a C₁₋₆ alkyl group, C₃₋₇ cycloalkyl group which may be substituted with C₁₋₆ alkyl, C₂₋₆ alkenyl group or C₂₋₆ alkynyl group, which comprises hydrolyzing under the presence of acid or alkali a compound represented by the formula (IIb): 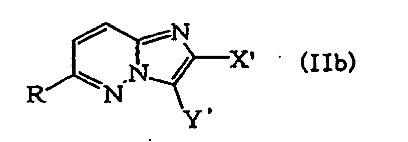 wherein X' and R are as defined above and Y' represents SO₂N=CH-NR¹R² (wherein R¹ and R² represent eachC₁₋₆ alkyl group, or R¹ and R² may be combined together with the adjacent nitrogen atom to form a heterocyclic ring), which is obtained by reacting an imidazo[1,2-b]pyridazine compound represented by the formula (Ib): 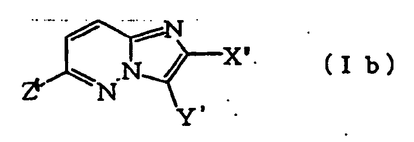 wherein X and Y' are as defined above, and Z' represents a halogen atom or OSO₂R³ (wherein R³ represents an optionally fluorinated C₁₋₆ alkyl group or phenyl group which may be substituted with C₁₋₆ alkyl), with one or more compounds selected from the organometallic compounds represented by the formula: 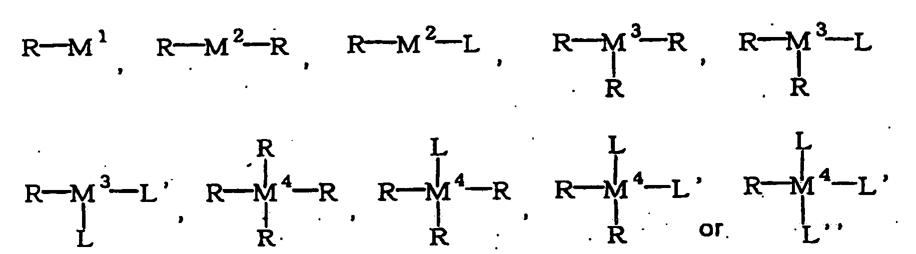 wherein R is as defined above, and M¹ represents an univalent metal, M² represents a divalent metal, M³ represents a trivalent metal and M⁴ represents a tetravalent metal, and L, L' and L" are the same or different and represent an anion, under the presence of transition metal catalyst.

## Patentansprüche

1. Verfahren zur Herstellung einer Verbindung, die durch die Formel (II) 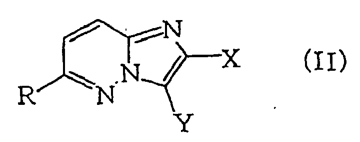 dargestellt wird, wobei X für ein Chloratom steht, Y für ein Wasserstoffatom oder SO₂N=CH-NR¹R² (wobei R¹ und R² jeweils für eine C₁-C₆-Alkylgruppe stehen oder R¹ und R² zusammen mit dem benachbarten Stickstoffatom unter Bildung eines heterocyclischen Rings kombiniert sein können) steht und R für eine C₁₋₆-Alkylgruppe, eine C₃₋₇-Cycloalkylgruppe, die mit C₁₋₆-Alkyl substituiert sein kann, eine C₂₋₆-Alkenylgruppe oder eine C₂₋₆-Alkinylgruppe steht, umfassend das Umsetzen einer Imidazo[1,2-b]pyridazin-Verbindung, die durch die Formel (I) 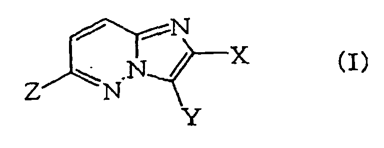 dargestellt wird, wobei X und Y wie oben definiert sind und Z für ein Chloratom steht, mit einer oder mehreren Verbindungen, die aus den metallorganischen Verbindungen ausgewählt sind, die durch die Formel 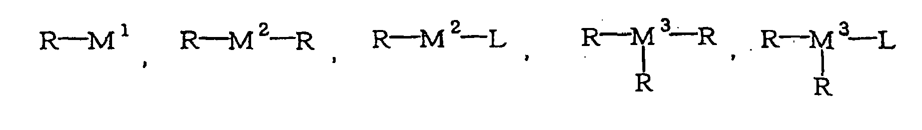 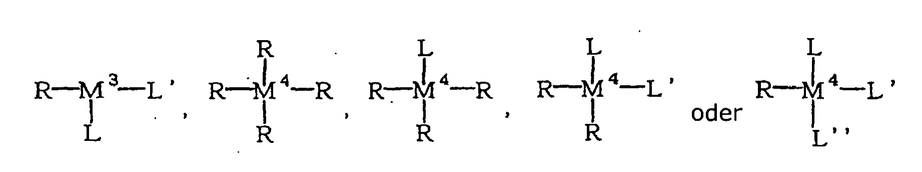 dargestellt werden, wobei R wie oben definiert ist und M¹ für ein einwertiges Metall steht, M² für ein zweiwertiges Metall steht, M³ für ein dreiwertiges Metall steht und M⁴ für ein vierwertiges Metall steht und L, L' und L" gleich oder verschieden sind und für ein Anion stehen, in Gegenwart eines Übergangsmetallkatalysators.

2. Verfahren gemäß Anspruch 1, wobei es sich bei dem Metall des Übergangsmetallkatalysators um Palladium, Nickel oder Eisen handelt.

3. Verfahren gemäß Anspruch 1, wobei es sich bei dem Metall des Übergangsmetallkatalysators um Nickel handelt.

4. Verfahren gemäß Anspruch 1, wobei es sich bei dem Metall der metallorganischen Verbindung um Magnesium oder Zink handelt.

5. Verfahren gemäß Anspruch 1, wobei R eine C₁₋₆-Alkylgruppe oder C₃₋₇-Cycloalkylgruppe, die mit C₁₋₆-Alkyl substituiert sein kann, ist.

6. Verfahren gemäß Anspruch 1, wobei Y ein Wasserstoffatom ist und R eine C₁₋₆-Alkylgruppe ist.

7. Verfahren gemäß Anspruch 3, wobei es sich bei dem Metall der metallorganischen Verbindung um Magnesium oder Zink handelt.

8. Verfahren gemäß Anspruch 7, wobei die metallorganische Verbindung ein C₁₋₆-Alkylmagnesiumhalogenid oder ein C₁₋₆-Alkylzinkhalogenid ist.

9. Verfahren gemäß Anspruch 8, wobei die metallorganische Verbindung ein Propylmagnesiumhalogenid oder Propylzinkhalogenid ist und es sich bei dem Nickelkatalysator um [1,3-Bis(diphenylphosphino)propan]nickel(II)-dichlorid oder Bis(triphenylphosphin)nickel(II)dichlorid handelt.

10. Verfahren zur Herstellung einer Sulfonamid-Verbindung, die durch die Formel (III) 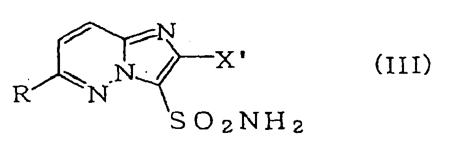 dargestellt wird, wobei X' für ein Halogenatom oder eine gegebenenfalls halogenierte C₁₋₆-Alkylgruppe steht und R für eine C₁₋₆-Alkylgruppe, eine C₃₋₇-Cycloalkylgruppe, die mit C₁₋₆-Alkyl substituiert sein kann, eine C₂₋₆-Alkenylgruppe oder eine C₂₋₆-Alkinylgruppe steht, umfassend das mit Chlorsulfonsäure erfolgende Sulfonieren einer Verbindung, die durch die Formel (IIa) 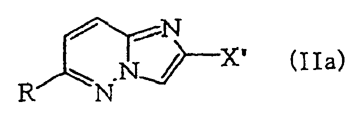 dargestellt wird, wobei X' und R wie oben definiert sind, und die durch Umsetzen einer Imidazo[1,2-b]pyridazin-Verbindung, die durch die Formel (Ia) 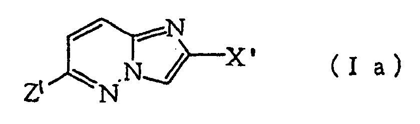 dargestellt wird, wobei X' wie oben definiert ist und Z' für ein Halogenatom oder OSO₂R³ (wobei R³ für eine gegebenenfalls fluorierte C₁₋₆-Alkylgruppe oder Phenylgruppe, die mit C₁₋₆-Alkyl substituiert sein kann) steht, mit einer oder mehreren Verbindungen, die aus den metallorganischen Verbindungen ausgewählt sind, die durch die Formel 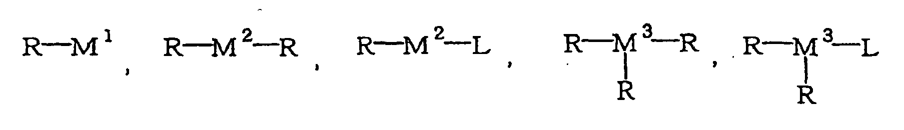 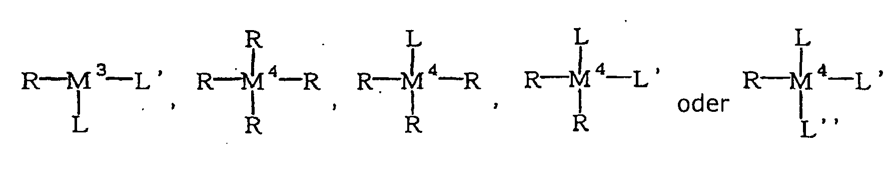 dargestellt werden, wobei R wie oben definiert ist und M¹ für ein einwertiges Metall steht, M² für ein zweiwertiges Metall steht, M³ für ein dreiwertiges Metall steht und M⁴ für ein vierwertiges Metall steht und L, L' und L" gleich oder verschieden sind und für ein Anion stehen, in Gegenwart eines Übergangsmetallkatalysators erhalten wird, und das anschließende Umwandeln in ein Sulfonylchlorid mit Phosphoroxidchlorid und dann das Umsetzen mit Ammoniak.

11. Verfahren zur Herstellung einer Sulfonamid-Verbindung, die durch die Formel (III) 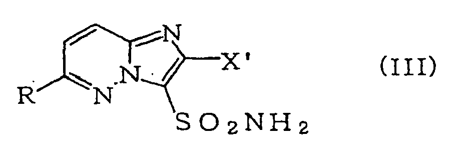 dargestellt wird, wobei X' für ein Halogenatom oder eine gegebenenfalls halogenierte C₁₋₆-Alkylgruppe steht und R für eine C₁₋₆-Alkylgruppe, eine C₃₋₇-Cycloalkylgruppe, die mit C₁₋₆-Alkyl substituiert sein kann, eine C₂₋₆-Alkenylgruppe oder eine C₂₋₆-Alkinylgruppe steht, umfassend das in Gegenwart von Säure oder Base erfolgende Hydrolysieren einer Verbindung, die durch die Formel (IIb) 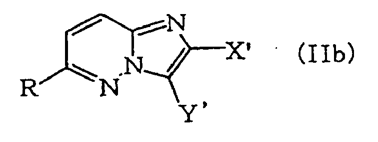 dargestellt wird, wobei X' und R wie oben definiert sind und Y' für SO₂N=CH-NR¹R² steht (wobei R¹ und R² jeweils für eine C₁-C₆-Alkylgruppe stehen oder R¹ und R² zusammen mit dem benachbarten Stickstoffatom unter Bildung eines heterocyclischen Rings kombiniert sein können), und die durch Umsetzen einer Imidazo[1,2-b]pyridazin-Verbindung, die durch die Formel Ia) 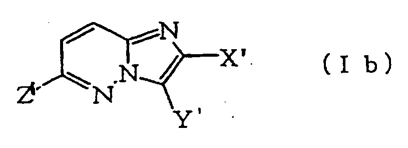 dargestellt wird, wobei X' und Y' wie oben definiert sind und Z' für ein Halogenatom oder OSO₂R³ (wobei R³ für eine gegebenenfalls fluorierte C₁₋₆-Alkylgruppe oder Phenylgruppe, die mit C₁₋₆-Alkyl substituiert sein kann) steht, mit einer oder mehreren Verbindungen, die aus den metallorganischen Verbindungen ausgewählt sind, die durch die Formel 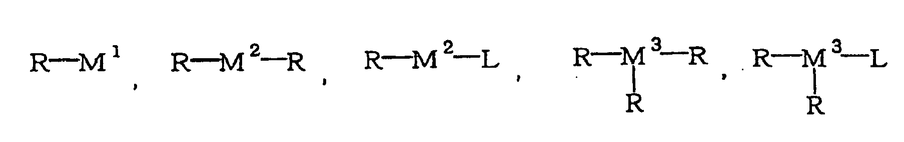 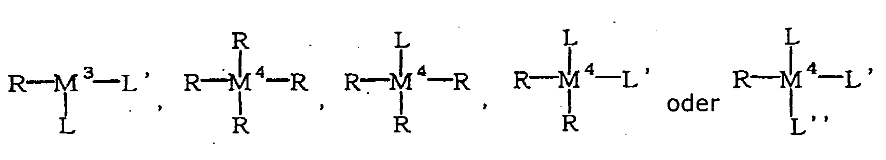 dargestellt werden, wobei R wie oben definiert ist und M¹ für ein einwertiges Metall steht, M² für ein zweiwertiges Metall steht, M³ für ein dreiwertiges Metall steht und M⁴ für ein vierwertiges Metall steht und L, L' und L" gleich oder verschieden sind und für ein Anion stehen, in Gegenwart eines Übergangsmetallkatalysators erhalten wird.

## Revendications

1. Procédé pour la préparation d'un composé représenté par la formule (II): 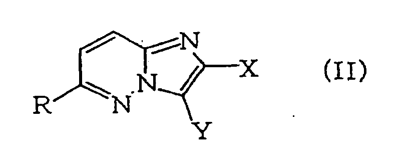 où X représente un atome de chlore, Y représente un atome d'hydrogène ou SO₂N=CH-NR¹R² (où R¹ et R² représentent chacun un groupe alkyle en C₁-C₆, ou R¹ et R² peuvent être combinés avec l'atome d'azote adjacent pour former un anneau hétérocyclique) et R représente un groupe alkyle en C₁₋₆, un groupe cycloalkyle en C₃₋₇ qui peut être substitué avec alkyle en C₁₋₆, un groupe alcényle en C₂₋₆ ou un groupe alcynyle en C₂₋₆, comprenant l'étape consistant à faire réagir un composé imidazo[1,2-b]pyridazine représenté par la formule (I): 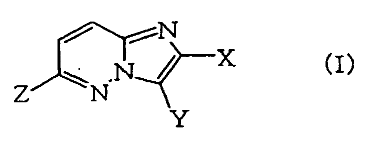 où X et Y sont tels que définis ci-dessus, et Z représente un atome de chlore, avec un ou plusieurs composés choisis parmi les composés organométalliques représentés par la formule: 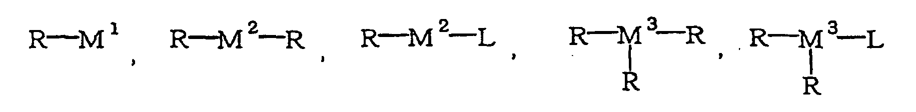 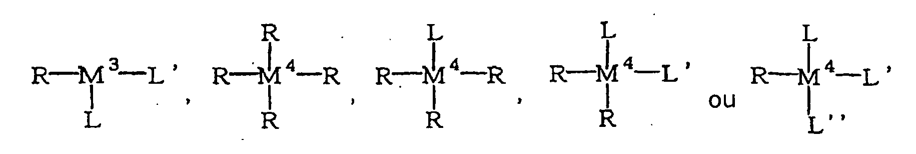 où R est tel que défini ci-dessus et M¹ représente un métal univalent, M² représente un métal divalent, M³ représente un métal trivalent et M⁴ représente un métal tétravalent, et L, L' et L" sont identiques ou différents et représentent un anion, en présence d'un catalyseur à base d'un métal de transition.

2. Procédé selon la revendication 1, dans lequel le métal du catalyseur à base d'un métal de transition est le palladium, le nickel ou le fer.

3. Procédé selon la revendication 1, dans lequel le métal du catalyseur à base d'un métal de transition est le nickel.

4. Procédé selon la revendication 1, dans lequel le métal du composé organométallique est le magnésium ou le zinc.

5. Procédé selon la revendication 1, dans lequel R est un groupe alkyle en C₁₋₆ ou un groupe cycloalkyle et C₃₋₇ qui peut être substitué avec alkyle en C₁₋₆.

6. Procédé selon la revendication 1, dans lequel Y est un atome d'hydrogène et R est un groupe alkyle en C₁₋₆.

7. Procédé selon la revendication 3, dans lequel le métal du composé organométallique est le magnésium ou le zinc.

8. Procédé selon la revendication 7, dans lequel le composé organométallique est un halogénure d'alkylmagnésium en C₁₋₆ ou un halogénure d'alkylzinc en C₁₋₆.

9. Procédé selon la revendication 8, dans lequel le composé organométallique est un halogénure de propylmagnésium ou un halogénure de propylzinc et le catalyseur à base de nickel est le dichlorure de [1,3-bis(diphényl-phosphino)propane]nickel(II) ou le dichlorure de bis(triphénylphosphine)-nickel(II).

10. Procédé pour la préparation d'un composé sulfonamide représenté par la formule (III): 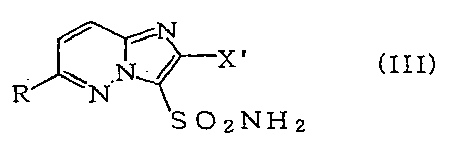 où X' représente un atome d'halogène ou un groupe alkyle en C₁-C₆ éventuellement halogéné et R représente un groupe alkyle en C₁₋₆, un groupe cycloalkyle en C₃₋₇ qui peut être substitué avec alkyle en C₁₋₆, un groupe alcényle en C₂₋₆ ou un groupe alcynyle en C₂₋₆, comprenant l'étape consistant à sulfonater avec l'acide chlorosulfonique un composé représenté par la formule (IIa) 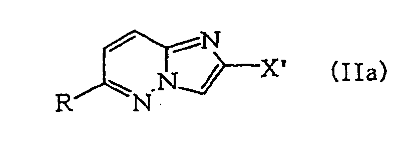 où X' et R sont tels que définis ci-dessus, qui est obtenu par la réaction d'un composé imidazo[1,2-b]pyridazine représenté par la formule (Ia): 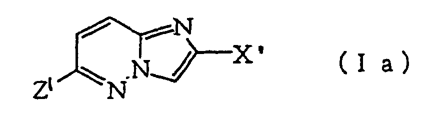 où X' est tel que défini ci-dessus, et Z' représente un atome d'halogène ou OSO₂R³ (où R³ représente un groupe alkyle en C₁-C₆ éventuellement fluoré ou un groupe phényle qui peut être substitué avec alkyle en C₁₋₆), avec un ou plusieurs composés choisis parmi les composés organométalliques représentés par la formule: 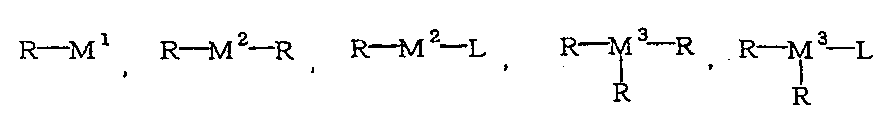 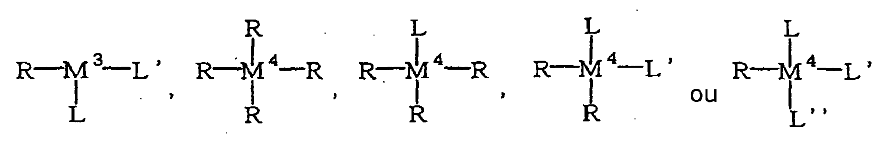 où R est tel que défini ci-dessus et M¹ représente un métal univalent, M² représente un métal divalent, M³ représente un métal trivalent et M⁴ représente un métal tétravalent, et L, L' et L" sont identiques ou différents et représentent un anion, en présence d'un catalyseur à base d'un métal de transition, puis convertir en un chlorure de sulfonyle avec l'oxychlorure de phosphore, puis faire réagir avec l'ammoniaque.

11. Procédé pour la préparation d'un composé sulfonamide représenté par la formule (III): 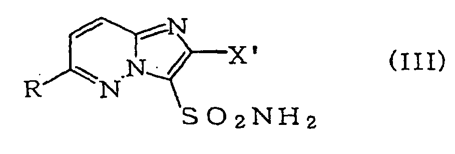 où X' représente un atome d'halogène ou un groupe alkyle en C₁-C₆ éventuellement halogéné et R représente un groupe alkyle en C₁₋₆, un groupe cycloalkyle en C₃₋₇ qui peut être substitué avec alkyle en C₁₋₆, un groupe alcényle en C₂₋₆ ou un groupe alcynyle en C₂₋₆, comprenant l'étape consistant à hydrolyser en présence d'un acide ou d'une base un composé représenté par la formule (IIb): 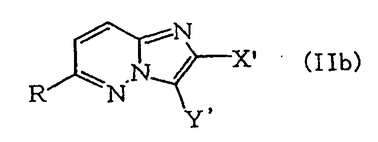 où X' et R sont tels que définis ci-dessus et Y' représente SO₂N=CH-NR¹R² (où R¹ et R² représentent chacun un groupe alkyle en C₁-C₆, ou R¹ et R² peuvent être combinés avec l'atome d'azote adjacent pour former un anneau hétérocyclique), qui est obtenu par la réaction d'un composé imidazo[1,2-b]pyridazine représenté par la formule (Ib): 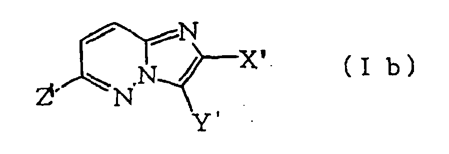 où X' et Y' sont tels que définis ci-dessus et Z' représente un atome d'halogène ou OSO₂R³ (où R³ représente un groupe alkyle en C₁-C₆ éventuellement fluoré ou un groupe phényle qui peut être substitué avec alkyle en C₁₋₆), avec un ou plusieurs composés choisis parmi les composés organométalliques représentés par la formule: 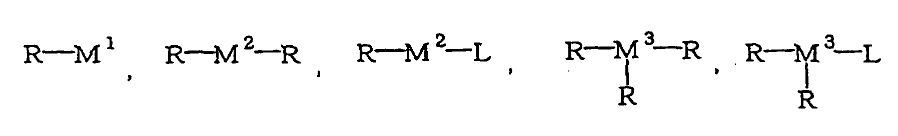 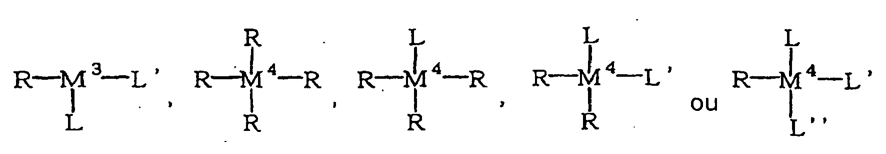 où R est tel que défini ci-dessus et M¹ représente un métal univalent, M² représente un métal divalent, M³ représente un métal trivalent et M⁴ représente un métal tétravalent, et L, L' et L" sont identiques ou différents et représentent un anion, en présence d'un catalyseur à base d'un métal de transition.
